# EUROPÄISCHE PATENTSCHRIFT

(11) **EP 3 121 179 B1**
(45) Veröffentlichungstag und Bekanntmachung des Hinweises auf die Patenterteilung: **17.10.2018**
(21) Anmeldenummer: 16180042.0
(22) Anmeldetag: 19.07.2016
(51) Int. Cl.: B01J 31/22, B01J 31/24, C07C 67/38, C07F 17/02, C07F 9/572, C07F 9/655, C07F 9/6553, C07F 15/00, C07F 9/58, C07F 9/6506

(54) **FERROCENBASIERTE VERBINDUNGEN UND DARAUF BASIERENDE PALLADIUM-KATALYSATOREN FÜR DIE ALKOXYCARBONYLIERUNG ETHYLENISCH UNGESÄTTIGTER VERBINDUNGEN**
FERROCENE-BASED COMPOUNDS AND PALLADIUM CATALYSTS BASED ON SAME FOR THE ALKOXYCARBONYLATION OF ETHYLENICALLY UNSATURATED COMPOUNDS
COMPOSÉS A BASE DE FERROCENE ET CATALYSEURS EN PALLADIUM A BASE DE CELLES-CI POUR L'ALKOXY-CARBONYLATION DE LIAISONS INSATUREES EN ETHYLENE

(30) Priorität: 23.07.2015 DE 102015213918
(43) Veröffentlichungstag der Anmeldung: 25.01.2017
(73) Patentinhaber: Evonik Degussa GmbH, 45128 Essen (DE)
(72) Erfinder: DONG, Kaiwu, 236800 Bo Zhou (CN); NEUMANN, Helfried, 18055 Rostock (DE); JACKSTELL, Ralf, 27478 Cuxhaven Altenwalde (DE); BELLER, Matthias, 18211 Ostseebad Nienhagen (DE); FRANKE, Robert, 45772 Marl (DE); HESS, Dieter, 45770 Marl (DE); DYBALLA, Katrin Marie, 45657 Recklinghausen (DE); FRIDAG, Dirk, 45721 Haltern am See (DE); GEILEN, Frank, 45721 Haltern am See (DE)

(56) Entgegenhaltungen:
- ALLOUCH FATIMA ET AL: "Ferrocenyl (P,N)-diphosphines incorporating pyrrolyl, imidazolyl or benzazaphospholyl moieties: Synthesis, coordination to group 10 metals and performances in palladium-catalyzed arylation reactions", JOURNAL OF ORGANOMETALLIC CHEMISTRY, ELSEVIER-SEQUOIA S.A. LAUSANNE, CH, Bd. 735, 23. März 2013 (2013-03-23), Seiten 38-46, XP028590941, ISSN: 0022-328X, DOI: 10.1016/J.JORGANCHEM.2013.03.025
- ANATOLIY P. MARCHENKO ET AL: "Stable N-Heterocyclic Carbenes: N-Alkyl-N'-phosphanylbenzimidazol-2-yliden es", EUROPEAN JOURNAL OF ORGANIC CHEMISTRY, Bd. 2012, Nr. 21, 12. Juli 2012 (2012-07-12), Seiten 4018-4033, XP55321595, DE ISSN: 1434-193X, DOI: 10.1002/ejoc.201200282
- DATABASE CAPLUS [Online] CHEMICAL ABSTRACTS SERVICE, COLUMBUS, OHIO, US; 2012, XP002765545, gefunden im STN Database accession no. 2012:1448458
- DATABASE CAPLUS [Online] CHEMICAL ABSTRACTS SERVICE, COLUMBUS, OHIO, US; 2000, XP002765546, gefunden im STN Database accession no. 2000:260134
- DATABASE CAPLUS [Online] CHEMICAL ABSTRACTS SERVICE, COLUMBUS, OHIO, US; 1975, XP002765547, gefunden im STN Database accession no. 1975:4356
- DATABASE CAPLUS [Online] CHEMICAL ABSTRACTS SERVICE, COLUMBUS, OHIO, US; 1974, XP002765548, gefunden im STN Database accession no. 1974:48110
- DATABASE CAPLUS [Online] CHEMICAL ABSTRACTS SERVICE, COLUMBUS, OHIO, US; 1973, XP002765549, gefunden im STN Database accession no. 1973:546600
- BIANCHINI C ET AL: "METHOXYCARBONYLATION OF ETHENE BY PALLADIUM(II) COMPLEXES WITH 1,1'-BIS(DIPHENYLPHOSPHINO)FERROCENE (DPPF) AND 1,1'-BIS(DIPHENYLPHOSPHINO)OCTAMETHYLFERRO CENE (DPPOMF)", ORGANOMETALLICS, AMERICAN CHEMICAL SOCIETY, US, Bd. 22, Nr. 12, 9. Juni 2003 (2003-06-09), Seiten 2409-2421, XP001164644, ISSN: 0276-7333, DOI: 10.1021/OM021049B

## Beschreibung

Die vorliegende Erfindung betrifft neue ferrocenbasierte Verbindungen und deren Verwendung in der Alkoxycarbonylierung.

Die Alkoxycarbonylierung ethylenisch ungesättigter Verbindungen ist ein Prozess mit steigender Bedeutung. Unter einer Alkoxycarbonylierung versteht man die Umsetzung von ethylenisch ungesättigten Verbindungen, wie Olefinen, mit Kohlenmonoxid und Alkoholen in Gegenwart eines Metalls bzw. eines Metallkomplexes und eines Liganden zu den entsprechenden Estern:

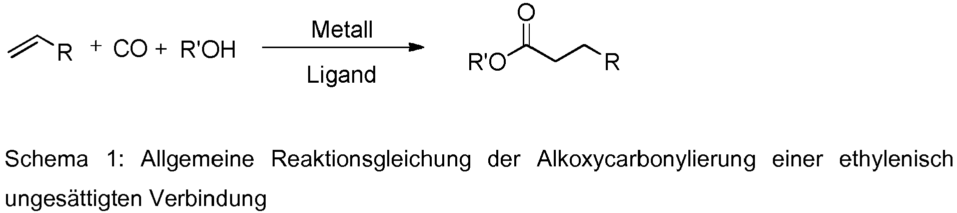

Unter den Alkoxycarbonylierungsreaktionen ist die Ethen-Methoxycarbonylierung zu 3-Methylpropionat von Bedeutung als Zwischenstufe für die Herstellung von Methylmethacrylat (S. G. Khokarale, E. J. García-Suárez, J. Xiong, U. V. Mentzel, R. Fehrmann, A. Riisager, Catalysis Communications 2014, 44, 73-75). Die Ethen-Methoxycarbonylierung wird in Methanol als Lösemittel bei milden Bedingungen mit einem durch Phosphinliganden modifizierten Palladiumkatalysator durchgeführt.

Ein sehr gutes katalytischen System wurde von Lucite - jetzt Mitsubishi Rayon - entwickelt und verwendet einen Liganden auf Basis von 1,2-Bis(di-tert-butylphosphinomethyl)benzol (DTBPMB) (W. Clegg, G. R. Eastham, M. R. J. Elsegood, R. P. Tooze, X. L. Wang, K. Whiston, Chem. Commun 1999, 1877-1878).

Anwendungen der Methoxycarbonylierung auf längerkettige Substrate sind z.B. in EP 0 662 467 beschrieben. Die Patentschrift beschreibt einen Prozess zur Herstellung von Dimethyladipat aus Methyl-3-Pentenoat. Als Pd-Quelle wird Pd(II)acetat verwendet. Als Beispiele geeigneter bidentater Phosphinliganden werden unter anderem 1,1'-Bis(diphenylphosphino)ferrocen, 1-(Diphenylphosphino)-1'-(diisopropylphosphino)ferrocen und 1,1'-Bis(isopropylphenylphosphino)ferrocen genannt. Die Liganden erzielen jedoch nur unzureichende Ausbeuten bei der Methoxycarbonylierung von Olefinen, insbesondere von langkettigen Olefinen wie 2-Octen und Di-n-Buten.

In BIANCHINI C. ET AL, "METHOXYCARBONYLATION OF ETHENE BY PALLADIUM(II) COMPLEXES WITH 1,1'-BIS(DIPHENYLPHOSPHINO)FERROCENE (DPPF) AND 1,1'-BIS(DIPHENYLPHOSPHINO)OCTAMETHYLFERROCENE (DPPOMF)", ORGANOMETALLICS, AMERICAN CHEMICAL SOCIETY, US, (20030609), Vol. 22, Nr. 12, Seiten 2409 - 2421 wird ein Verfahren zur Methoxycarbonylierung von Ethen beschrieben. Als Katalysator kommt ein Pd-Komplex mit Ferrocenyl-Liganden zum Einsatz.

Die technische Aufgabe, welche der vorliegenden Erfindung zugrunde lag, ist die Bereitstellung neuer ferrocenbasierter Verbindungen als Liganden für Alkoxycarbonylierungsreaktionen. Diese Verbindungen sollen insbesondere bei der Alkoxycarbonylierung von Ethen und langkettigen Olefinen wie Di-n-Buten verbesserte Ausbeuten erzielen. Insbesondere soll bei der Alkoxycarbonylierungsreaktion die Raum-ZeitAusbeute gesteigert werden.

Diese Aufgabe wird gelöst durch Diphosphinverbindungen gemäß Formel (I) 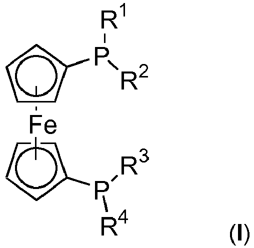 wobei
R¹ und R³ jeweils einen Heteroarylrest mit fünf Ringatomen darstellen,
R² und R⁴ jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl;
R¹ und R³ jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl, -CONH-(C₃-C₁₂)-Cycloalkyl,-CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können;
und R² und R⁴, falls diese für -(C₁-C₁₂)-Alkyl, -(C₃-C₁₂)-Cycloalkyl, -(C₃-C₁₂)-Heterocycloalkyl oder -(C₆-C₂₀)-Aryl stehen,
jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl, -CONH-(C₃-C₁₂)-Cycloalkyl,-CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können;
ausgenommen die Verbindungen gemäß den Formeln (1) und (2)

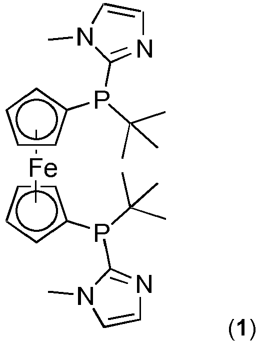

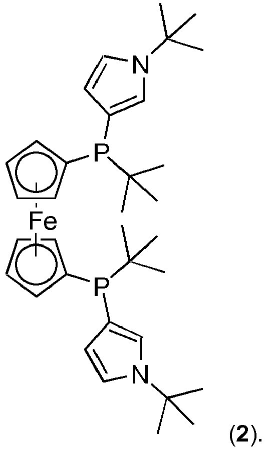

Die erfindungsgemäßen Verbindungen eignen sich als zweizähnige Phosphinliganden für Pd-Komplexe, mit denen sich bei der Alkoxycarbonylierung einer Vielzahl ethylenisch ungesättigter Verbindungen hohe Ausbeuten erzielen lassen. Insbesondere eignen sich die erfindungsgemäßen Verbindungen zur Alkoxycarbonylierung von Ethen und langkettigen Olefinen wie Di-n-Buten.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, n-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, *n*-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Die Erläuterungen zum Begriff (C₁-C₁₂)-Alkyl gelten insbesondere auch für die Alkylgruppen in -O-(C₁-C₁₂)-Alkyl, -S-(C₁-C₁₂)-Alkyl, -COO-(C₁-C₁₂)-Alkyl, -CONH-(C₁-C₁₂)-Alkyl, -CO-(C₁-C₁₂)-Alkyl und -N-[(C₁-C₁₂)-Alkyl]₂.

Der Begriff (C₃-C₁₂)-Cycloalkyl umfasst mono-, bi- oder tricyclische Kohlenwasserstoffgruppen mit 3 bis 12 Kohlenstoffatomen. Vorzugsweise handelt es sich dabei um (C₅-C₁₂)-Cycloalkyl.

Die (C₃-C₁₂)-Cycloalkylgruppen weisen vorzugsweise 3 bis 8, besonders bevorzugt 5 oder 6, Ringatome auf.

Geeignete (C₃-C₁₂)-Cycloalkylgruppen sind insbesondere Cyclopropyl, Cyclobutyl, Cyclopentyl, Cyclohexyl, Cycloheptyl, Cyclooctyl, Cyclododecyl, Cyclopentadecyl, Norbonyl, Adamantyl.

Die Erläuterungen zum Begriff (C₃-C₁₂)-Cycloalkyl gelten insbesondere auch für die Cycloalkylgruppen in -O-(C₃-C₁₂)-Cycloalkyl, -S-(C₃-C₁₂)-Cycloalkyl, -COO-(C₃-C₁₂)-Cycloalkyl, -CONH-(C₃-C₁₂)-Cycloalkyl, -CO-(C₃-C₁₂)-Cycloalkyl.

Der Begriff (C₃-C₁₂)-Heterocycloalkyl umfasst nichtaromatische, gesättigte oder teilweise ungesättigte cycloaliphatische Gruppen mit 3 bis 12 Kohlenstoffatomen, wobei eins oder mehrere der Ringkohlenstoffatome durch Heteroatome ersetzt sind. Die (C₃-C₁₂)-Heterocycloalkylgruppen weisen vorzugsweise 3 bis 8, besonders bevorzugt 5 oder 6, Ringatome auf und sind ggf. mit aliphatischen Seitenketten substituiert. In den Heterocycloalkylgruppen sind im Unterschied zu den Cycloalkylgruppen eins oder mehrere der Ringkohlenstoffatome durch Heteroatome oder heteroatomhaltige Gruppen ersetzt. Die Heteroatome oder die heteroatomhaltigen Gruppen sind vorzugsweise ausgewählt unter O, S, N, N(=O), C(=O), S(=O). Eine (C₃-C₁₂)-Heterocycloalkylgruppe im Sinne dieser Erfindung ist somit auch Ethylenoxid.

Geeignete (C₃-C₁₂)-Heterocycloalkylgruppen sind insbesondere Tetrahydrothiophenyl, Tetrahydrofuryl, Tetrahydropyranyl und Dioxanyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

Der Begriff (C₃-C₂₀)-Heteroaryl umfasst mono- oder polycyclische aromatische Kohlenwasserstoffreste mit 3 bis 20 Kohlenstoffatomen, wobei eins oder mehrere der Kohlenstoffatome durch Heteroatome ersetzt sind. Bevorzugte Heteroatome sind N, O und S. Die (C₃-C₂₀)-Heteroarylgruppen weisen 3 bis 20, bevorzugt 6 bis 14, besonders bevorzugt 6 bis 10 Ringatome auf. Somit ist beispielsweise Pyridyl im Rahmen dieser Erfindung ein C₆-Heteroarylrest, Furyl ist ein C₅-Heteroarylrest.

Geeignete (C₃-C₂₀)-Heteroarylgruppen sind insbesondere Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

Der Begriff (C₃-C₂₀)-Heteroaryl umfasst auch Heteroarylreste mit fünf Ringatomen.

Geeignete Heteroarylreste mit fünf Ringatomen sind insbesondere Furyl, Thienyl, Pyrolyl, Imidazolyl, Oxazolyl, Isoxazolyl, Thiazolyl, Isothiazolyl, Pyrazolyl, Furazanyl, Tetrazolyl.

Der Begriff Halogen umfasst insbesondere Fluor, Chlor, Brom und lod. Besonders bevorzugt sind Fluor und Chlor.
In einer Ausführungsform können die Reste R¹ und R³ jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein.

In einer Ausführungsform können die Reste R¹ und R³ jeweils unabhängig voneinander mit einem oder mehreren Substituenten ausgewählt aus -(C₁-C₁₂)-Alkyl, -(C₃-C₁₂)-Cycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl substituiert sein.

In einer Ausführungsform können die Reste R¹ und R³ jeweils unabhängig voneinander mit einem oder mehreren Substituenten ausgewählt aus -(C₁-C₁₂)-Alkyl, -(C₃-C₁₂)-Cycloalkyl, -O-(C₁-C₁₂)-Alkyl, -O-(C₃-C₁₂)-Cycloalkyl, -(C₆-C₂₀)-Aryl substituiert sein.

In einer Ausführungsform können die Reste R¹ und R³ jeweils unabhängig voneinander mit einem oder mehreren Substituenten ausgewählt aus -(C₁-C₁₂)-Alkyl substituiert sein.

In einer Ausführungsform sind die Reste R¹ und R³ unsubstituiert.

In einer Ausführungsform können die Reste R² und R⁴, falls sie für -(C₁-C₁₂)-Alkyl, -(C₃-C₁₂)-Cycloalkyl, -(C₃-C₁₂)-Heterocycloalkyl oder -(C₆-C₂₀)-Aryl stehen, jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein.

In einer Ausführungsform können die Reste R² und R⁴, falls sie für -(C₁-C₁₂)-Alkyl, -(C₃-C₁₂)-Cycloalkyl, -(C₃-C₁₂)-Heterocycloalkyl oder -(C₆-C₂₀)-Aryl stehen, jeweils unabhängig voneinander mit einem oder mehreren Substituenten ausgewählt aus -(C₁-C₁₂)-Alkyl, -(C₃-C₁₂)-Cycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl substituiert sein.

In einer Ausführungsform können die Reste R² und R⁴, falls sie für -(C₁-C₁₂)-Alkyl, -(C₃-C₁₂)-Cycloalkyl, -(C₃-C₁₂)-Heterocycloalkyl oder -(C₆-C₂₀)-Aryl stehen, jeweils unabhängig voneinander mit einem oder mehreren Substituenten ausgewählt aus -(C₁-C₁₂)-Alkyl, -(C₃-C₁₂)-Cycloalkyl, -O-(C₁-C₁₂)-Alkyl, -O-(C₃-C₁₂)-Cycloalkyl, -(C₆-C₂₀)-Aryl substituiert sein.

In einer Ausführungsform können die Reste R² und R⁴, falls sie für -(C₁-C₁₂)-Alkyl, -(C₃-C₁₂)-Cycloalkyl, -(C₃-C₁₂)-Heterocycloalkyl oder -(C₆-C₂₀)-Aryl stehen, jeweils unabhängig voneinander mit einem oder mehreren Substituenten ausgewählt aus -(C₁-C₁₂)-Alkyl substituiert sein.

In einer Ausführungsform sind die Reste R² und R⁴ unsubstituiert.

In einer Ausführungsform sind R¹ und R³ jeweils unabhängig voneinander ausgewählt aus Furyl, Thienyl, Pyrrolyl, Imidazolyl, Oxazolyl, Isoxazolyl, Thiazolyl, Isothiazolyl, Pyrazolyl, Furazanyl, Tetrazolyl; bevorzugt aus Furyl, Thienyl, 2-Pyrrolyl, 4-Imidazolyl, Oxazolyl, Isoxazolyl, Thiazolyl, Isothiazolyl, Pyrazolyl, Furazanyl, Tetrazolyl. R¹ und R³ können dabei wie oben beschrieben substituiert sein.

In einer Ausführungsform sind R¹ und R³ jeweils unabhängig voneinander ausgewählt aus 2-Furyl, 3-Furyl, 2-Thienyl, 3-Thienyl, 2-Pyrrolyl, 4-Imidazolyl. R¹ und R³ können dabei wie oben beschrieben substituiert sein.

In einer besonders bevorzugten Ausführungsform sind R¹ und R³ jeweils unabhängig voneinander ausgewählt aus Furyl und Thienyl, insbesondere 2-Furyl und 2-Thienyl. R¹ und R³ können dabei wie oben beschrieben substituiert sein.

In einer Ausführungsform sind R² und R⁴ jeweils unabhängig voneinander ausgewählt aus-(C₁-C₁₂)-Alkyl, -(C₃-C₁₂)-Cycloalkyl und -(C₆-C₂₀)-Aryl. R² und R⁴ können dabei wie oben beschrieben substituiert sein.

In einer Ausführungsform sind R² und R⁴ jeweils unabhängig voneinander ausgewählt aus-(C₁-C₁₂)-Alkyl. R² und R⁴ können dabei wie oben beschrieben substituiert sein.

In einer Ausführungsform stehen die Reste R¹ und R³ jeweils für einen Heteroarylrest mit fünf Ringatomen ausgewählt aus Furyl und Thienyl, und die Reste R² und R⁴ stehen jeweils für-(C₁-C₁₂)-Alkyl;
wobei
R¹, R², R³, R⁴ jeweils unabhängig voneinander mit einem oder mehreren der oben beschriebenen Substituenten substituiert sein können.

In einer Ausführungsform weist die Verbindung eine Struktur gemäß einer der Formeln (**16**), (**22**) und (**34**) auf:

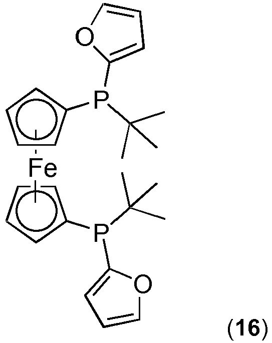

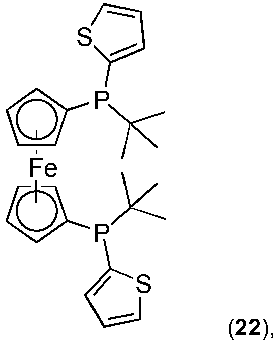

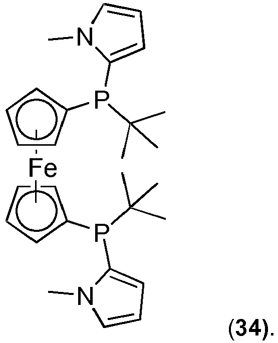

Die erfindungsgemäßen Diphosphinverbindungen können beispielsweise durch Umsetzung von Ferrocen mit Butyllithium und einer Chlorphosphinverbindung gewonnen werden.

Die Erfindung betrifft weiterhin Komplexe umfassend Pd und eine erfindungsgemäße Diphosphinverbindung. Bei diesen Komplexen dient die erfindungsgemäße Diphosphinverbindung als zweizähniger Ligand für das Metallatom. Die Komplexe dienen beispielsweise als Katalysatoren für die Alkoxycarbonylierung. Mit den erfindungsgemäßen Komplexen lassen sich hohe Ausbeuten bei der Alkoxycarbonylierung einer Vielzahl unterschiedlicher ethylenisch ungesättigter Verbindungen erzielen.

Die erfindungsgemäßen Komplexe können außerdem weitere Liganden umfassen, die an das Metallatom koordinieren. Dabei handelt es sich beispielsweise um ethylenisch ungesättigte Verbindungen oder Anionen. Geeignete zusätzliche Liganden sind beispielsweise Styrol, Acetat-Anionen, Maleinimide (z.B. N-Methylmaleinimid), 1,4-Naphthochinon, Trifluoroacetat-Anionen oder Chloridanionen.

Die Erfindung betrifft weiterhin die Verwendung einer erfindungsgemäßen Diphosphinverbindung zur Katalyse einer Alkoxycarbonylierungsreaktion. Dabei kann die erfindungsgemäße Verbindung insbesondere als erfindungsgemäßer Metallkomplex eingesetzt werden.

Die Erfindung betrifft außerdem ein Verfahren umfassend die Verfahrensschritte:
a) Vorlegen einer ethylenisch ungesättigten Verbindung;
b) Zugabe einer erfindungsgemäßen Diphosphinverbindung und einer Verbindung, welche Pd umfasst,
   oder Zugabe eines erfindungsgemäßen Komplexes umfassend Pd und eine erfindungsgemäße Diphosphinverbindung;
c) Zugabe eines Alkohols;
d) Zuführen von CO;
e) Erwärmen des Reaktionsgemisches, wobei die ethylenisch ungesättigte Verbindung zu einem Ester umgesetzt wird.

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis c) vorgelegt wurden. Die Schritte d) und e) können gleichzeitig oder nacheinander erfolgen. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch im Folgenden zur Vereinfachung als Olefine bezeichnet. Die Doppelbindungen können terminal oder intern sein.

Bevorzugt sind ethylenisch ungesättigte Verbindungen mit 2 bis 30 Kohlenstoffatomen, bevorzugt 2 bis 22 Kohlenstoffatomen, besonders bevorzugt 2 bis 12 Kohlenstoffatomen.

In einer Ausführungsform umfasst die ethylenisch ungesättigte Verbindung 4 bis 30 Kohlenstoffatome, bevorzugt 6 bis 22 Kohlenstoffatome, besonders bevorzugt 8 bis 12 Kohlenstoffatome, am meisten bevorzugt 8 Kohlenstoffatome.

Die ethylenisch ungesättigten Verbindungen können zusätzlich zu der einen oder mehreren Doppelbindungen weitere funktionelle Gruppen enthalten. Vorzugsweise umfasst die ethylenisch ungesättigte Verbindung eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl- , Amino-, Ether-, Thioether-, Aryl-, Heteroaryl- oder Silylgruppen und/oder Halogensubstituenten. Dabei umfasst die ethylenisch ungesättigte Verbindung vorzugsweise insgesamt 2 bis 30 Kohlenstoffatome, bevorzugt 2 bis 22 Kohlenstoffatome, besonders bevorzugt 2 bis 12 Kohlenstoffatome.

In einer Ausführungsform umfasst die ethylenisch ungesättigte Verbindung keine weiteren funktionellen Gruppen außer Kohlenstoff-Kohlenstoff-Doppelbindungen.

In einer besonders bevorzugten Ausführungsform handelt es sich bei der ethylenisch ungesättigten Verbindung um ein unfunktionalisiertes Alken mit mindestens einer Doppelbindung und 2 bis 30 Kohlenstoffatomen, bevorzugt 6 bis 22 Kohlenstoffatomen, weiter bevorzugt 8 bis 12 Kohlenstoffatomen, am meisten bevorzugt 8 Kohlenstoffatomen.

Geeignete ethylenisch ungesättigte Verbindungen sind beispielsweise:
Ethen;
Propen;
C4-Olefine, wie 1-Buten, cis-2-Buten, trans-2-Buten, Gemisch von cis- und trans-2-Buten, Isobuten, 1,3-Butadien; Raffinat I bis III, Crack-C4
C5-Olefine, wie 1-Penten, 2-Penten, 2-Methyl-1-buten, 2-Methyl-2-buten, 2-Methyl-1,3-butadien (Isopren), 1,3-Pentadien;
C6-Olefine, wie Tetramethylethylen, 1,3-Hexadien, 1,3-Cyclohexadien;
C7-Olefine, wie 1-Methylcyclohexen, 2,4-Heptadien, Norbonadien;
C8-Olefine, wie 1-Octen, 2-Octen, Cycloocten, Di-n-Buten, Di-iso-buten, 1,5-Cyclooctadien, 1,7-Octadien;
C9-Olefine, wie Tripropen;
C10-Olefine, wie Dicylcopentadien;
Undecene;
Dodecene;
interne C14-Olefine;
interne C15- bis C18-Olefine;
lineare oder verzweigte, cyclische, acyclische oder teilweise cyclische, interne C15- bis C30-Olefine;
Triisobuten, Tri-n-buten;
Terpene, wie Limonen, Geraniol, Farnesol, Pinen, Myrcen, Carvon, 3-Caren;
mehrfach ungesättigte Verbindungen mit 18 Kohlenstoffatomen, wie Linolsäure oder Linolensäure;
Ester ungesättigter Carbonsäuren, wie Vinylester von Essig- oder Propansäure, Alkylester ungesättigter Carbonsäuren, Methyl- oder Ethylester von Acrylsäure oder Methacrylsäure, Ölsäureester, Ölsäure Methyl- oder Ethylester, Ester der Linol- oder Linolensäure;
Vinylverbindungen, wie Vinylacetat, Vinylcyclohexen, Styrol, alpha-Methylstyrol, 2-Isopropenylnaphthalin;
2-Methyl-2-pentenal, Methyl-3-Pentenoat, Methacrylanhydrid.

In einer Variante des Verfahrens ist die ethylenisch ungesättigte Verbindung ausgewählt aus Propen, 1-Buten, cis- und/oder trans-2-Buten, oder Mischungen davon.

In einer Variante des Verfahrens ist die ethylenisch ungesättigte Verbindung ausgewählt aus 1-Penten, cis- und/oder trans-2-Penten, 2-Methyl-1-buten, 2-Methyl-2-buten, 3-Methyl-1-buten, oder Mischungen davon.

In einer bevorzugten Ausführungsform ist die ethylenisch ungesättigte Verbindung ausgewählt aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, n-Octen, 1-Octen, 2-Octen, oder Mischungen davon.

In einer Variante wird ein Gemisch ethylenisch ungesättigter Verbindungen eingesetzt. Als Gemisch wird im Sinne dieser Erfindung eine Zusammensetzung bezeichnet, die wenigsten zwei verschiedene ethylenisch ungesättigte Verbindungen enthält, wobei der Anteil jeder einzelnen ethylenisch ungesättigten Verbindungen vorzugsweise wenigstens 5 Gewichts-% bezogen auf das Gesamtgewicht des Gemisches beträgt.

Vorzugsweise wird ein Gemisch ethylenisch ungesättigter Verbindungen mit jeweils 2 bis 30 Kohlenstoffatomen, bevorzugt 4 bis 22 Kohlenstoffatomen, besonders bevorzugt 6 bis 12 Kohlenstoffatomen, am meisten bevorzugt 8 bis 10 Kohlenstoffatomen eingesetzt.

Geeignete Gemische ethylenisch ungesättigter Verbindungen sind die sogenannten Raffinate I bis III. Raffinat I umfasst 40 bis 50 % iso-Buten, 20 bis 30 % 1-Buten, 10 bis 20 % cis- und trans-2-Buten, bis zu 1 % 1,3-Butadien und 10 bis 20 % n-Butan und Isobutan. Das Raffinat II ist ein Teil der beim Naphthacracken entstehenden C₄-Fraktion und besteht im Wesentlichen aus den isomeren n-Butenen, Isobutan und n-Butan nach Abtrennung von Isobuten aus Raffinat I. Raffinat III ist ein Teil der beim Naphthacracken entstehenden C₄-Fraktion und besteht im Wesentlichen aus den isomeren n-Butenen und n-Butan.

Ein weiteres geeignetes Gemisch ist Di-n-Buten, auch bezeichnet als Dibuten, DNB oder DnB. Di-n-Buten ist ein Isomerengemisch von C8-Olefinen, das aus der Dimerisierung von Gemischen aus 1-Buten, cis-2-Buten und trans-2-Buten entsteht. Technisch werden der Regel Raffinat II oder Raffinat III-Ströme einer katalytischen Oligomerisierung unterworfen, wobei die enthaltenen Butane (n/iso) unverändert hervorgehen und die enthaltenen Olefine ganz oder teilweise umgesetzt werden. Neben dem dimeren Di-n-Buten, entstehen in der Regel auch höhere Oligomere (Tributen C12, Tetrabuten C16), die nach der Reaktion destillativ abgetrennt werden. Diese können ebenfalls als Edukte eingesetzt werden.

In einer bevorzugten Variante wird ein Gemisch umfassend iso-Buten, 1-Buten, cis- und trans-2-Buten eingesetzt. Vorzugsweise umfasst das Gemisch 1-Buten, cis- und trans-2-Buten.

Die erfindungsgemäße Alkoxycarbonylierung wird durch den erfindungsgemäßen Pd-Komplex katalysiert. Der Pd-Komplex kann dabei in Verfahrensschritt b) entweder als präformierter Komplex umfassend Pd und den erfindungsgemäßen Phosphinliganden zugegeben werden, oder *in situ* aus einer Verbindung, welche Pd umfasst, und dem freien Phosphinliganden gebildet werden. Dabei wird die Verbindung, welche Pd umfasst, auch als Katalysatorvorstufe bezeichnet.

In dem Fall, dass der Katalysator *in situ* gebildet wird, kann der Ligand im Überschuss zugegeben werden, so dass im Reaktionsgemisch auch ungebundener Ligand vorliegt.

Auch im Falle des Komplexes, der gleich zu Beginn zugesetzt wird, kann auch weiterer Ligand zugegeben werden, so dass im Reaktionsgemisch auch ungebundener Ligand vorliegt.

In einer Variante ist die Verbindung, welche Pd umfasst, ausgewählt aus Palladiumdichlorid (PdCl₂), Palladium(II)-acetylacetonat [Pd(acac)₂], Palladium(II)-acetat [Pd(OAc)₂], Dichloro(1,5-cyclooctadiene)palladium(II) [Pd(cod)₂Cl₂], Bis(dibenzylideneaceton)palladium [Pd(dba)₂], Bis(acetonitrile)dichloropalladium(II) [Pd(CH₃CN)₂Cl₂], Palladium(cinnamyl)dichlorid [Pd(cinnamyl)Cl₂].

Vorzugsweise handelt es sich bei der Verbindung, welche Pd umfasst, um PdCl₂, Pd(acac)₂ oder Pd(OAc)₂. Besonders geeignet ist PdCl₂.

Der Alkohol im Verfahrensschritt c) kann verzweigt oder linear sein, cyclisch, alicyclisch, teilweise zyklisch oder aliphatisch und stellt insbesondere ein C₁- bis C₃₀-Alkanol dar. Es können Monoalkohole oder Polyalkohole verwendet werden.

Der Alkohol in Verfahrensschritt c) umfasst vorzugsweise 1 bis 30 Kohlenstoffatome, bevorzugt 1 bis 22 Kohlenstoffatome, besonders bevorzugt 1 bis 12 Kohlenstoffatome. Es kann sich dabei um einen Monoalkohol oder einen Polyalkohol handeln.

Der Alkohol kann zusätzlich zu der einen oder mehreren Hydroxylgruppen weitere funktionelle Gruppen enthalten. Vorzugsweise kann der Alkohol zusätzlich eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Sulfhydryl- , Amino-, Ether-, Thioether-, Aryl-, Heteroaryl- oder Silylgruppen, und/oder Halogensubstituenten enthalten.

In einer Ausführungsform umfasst der Alkohol keine weiteren funktionellen Gruppen außer Hydroxylgruppen.

Der Alkohol kann ungesättigte und aromatische Gruppen enthalten. Vorzugsweise handelt es sich jedoch um einen aliphatischen Alkohol.

Als aliphatischer Alkohol wird im Rahmen dieser Erfindung ein Alkohol bezeichnet, der keine aromatischen Gruppen umfasst, also beispielsweise ein Alkanol, Alkenol oder Alkinol.

In einer Ausführungsform handelt es sich bei dem Alkohol um ein Alkanol mit einer oder mehrerer Hydroxylgruppen und 1 bis 30 Kohlenstoffatomen, bevorzugt 1 bis 22 Kohlenstoffatomen, besonders bevorzugt 1 bis 12 Kohlenstoffatomen, am meisten bevorzugt 1 bis 6 Kohlenstoffatomen.

In einer Variante des Verfahrens ist der Alkohol im Verfahrensschritt c) ausgewählt aus der Gruppe der Monoalkohole.

In einer Variante des Verfahrens ist der Alkohol im Verfahrensschritt c) ausgewählt aus: Methanol, Ethanol, 1-Propanol, Iso-Propanol, Iso-Butanol, tert-Butanol, 1-Butanol, 2-Butanol, 1-Pentanol, 2-Pentanol, 3-Pentanol, 1-Hexanol, Cyclohexanol, Phenol, 2-Ethylhexanol, Isononanol, 2-Propylheptanol.

In einer bevorzugten Variante ist der Alkohol in Verfahrensschritt c) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

In einer Variante des Verfahrens ist der Alkohol im Verfahrensschritt c) ausgewählt aus der Gruppe der Polyalkohole.

In einer Variante des Verfahrens ist der Alkohol im Verfahrensschritt c) ausgewählt aus: Diolen, Triolen, Tetraolen.

In einer Variante des Verfahrens ist der Alkohol im Verfahrensschritt c) ausgewählt aus: Cyclohexan-1,2-diol, 1,2-Ethandiol, 1,3-Propandiol, Glycerol, 1,2,4-Butantriol,2-Hydroxymethyl-1,3-Propandiol, 1,2,6-Trihydroxyhexan, Pentaerythritol, 1,1,1-Tri(hydroxymethyl)ethan, Brenzkatechin, Resorcin und Hydroxyhydrochinon.

In einer Variante des Verfahrens ist der Alkohol im Verfahrensschritt c) ausgewählt aus: Sucrose, Fructose, Mannose, Sorbose, Galactose und Glucose.

In einer bevorzugten Variante des Verfahrens ist der Alkohol im Verfahrensschritt c) ausgewählt aus Methanol, Ethanol, 1-Propanol, 1-Butanol, 1-Pentanol, 1-Hexanol.

In einer besonders bevorzugten Variante des Verfahrens ist der Alkohol im Verfahrensschritt c) ausgewählt aus: Methanol, Ethanol.

In einer besonders bevorzugten Variante des Verfahrens ist der Alkohol im Verfahrensschritt c) Methanol.

In einer Variante des Verfahrens wird der Alkohol im Verfahrensschritt c) im Überschuss eingesetzt.

In einer Variante des Verfahrens wird der Alkohol im Verfahrensschritt c) gleichzeitig als Lösungsmittel eingesetzt.

In einer Variante des Verfahrens wird ein weiteres Lösungsmittel verwendet, ausgewählt aus: Toluol, Xylol, Tetrahydrofuran (THF) oder Methylenchlorid (CH₂Cl₂).

CO wird in Schritt d) vorzugsweise bei einem CO-Partialdruck zwischen 0,1 und 10 MPa (1 bis 100 bar), bevorzugt zwischen 1 und 8 MPa (10 bis 80 bar), besonders bevorzugt zwischen 2 und 4 MPa (20 bis 40 bar) zugeführt.

Die Reaktionsmischung wird Schritt e) des erfindungsgemäßen Verfahrens vorzugsweise auf eine Temperatur zwischen 10 °C und 180 °C, bevorzugt zwischen 20 und 160 °C, besonders bevorzugt zwischen 40 und 120 °C erwärmt, um die ethylenisch ungesättigte Verbindung zu einem Ester umzusetzen.

Das molare Verhältnis von der in Schritt a) vorgelegten ethylenisch ungesättigten Verbindung zu dem in Schritt c) zugegebenen Alkohol beträgt vorzugsweise zwischen 1:1 bis 1:20, bevorzugt 1:2 bis 1:10, besonders bevorzugt 1:3 bis 1:4.

Das Massenverhältnis von Pd zu der in Schritt a) vorgelegten ethylenisch ungesättigten Verbindung beträgt vorzugsweise zwischen 0,001 und 0,5 Gew.-%, bevorzugt zwischen 0,01 und 0,1 Gew.-%, besonders bevorzugt zwischen 0,01 und 0,05 Gew.-%.

Das molare Verhältnis der erfindungsgemäßen Diphosphinverbindung zu Pd beträgt vorzugsweise zwischen 0,1:1 und 400:1, bevorzugt zwischen 0,5:1 und 400:1, besonders bevorzugt zwischen 1:1 und 100:1, am meisten bevorzugt zwischen 2:1 und 50:1.

Vorzugsweise wird das Verfahren unter Zusatz einer Säure durchgeführt. In einer Variante umfasst das Verfahren deshalb zusätzlich den Schritt c'): Zugabe einer Säure zum Reaktionsgemisch. Dabei kann es sich vorzugsweise um eine Brønsted- oder eine Lewis-Säure handeln.

Geeignete Brønsted-Säuren haben vorzugsweise eine Säurestärke von pKₛ ≤ 5, bevorzugt eine Säurestärke von pKₛ ≤ 3. Die angegebene Säurestärke pKₛ bezieht sich auf den bei Normalbedingungen (25°C, 1,01325 bar) bestimmten pKₛ-Wert. Bei einer mehrprotonigen Säure bezieht sich die Säurestärke pKₛ im Rahmen dieser Erfindung auf den pKₛ-Wert des ersten Protolyseschrittes.

Vorzugsweise ist die Säure keine Carbonsäure.

Geeignete Brønsted-Säuren sind beispielsweise Perchlorsäure, Schwefelsäure, Phosphorsäure, Methylphosphonsäure und Sulfonsäuren. Vorzugsweise handelt es sich bei der Säure um Schwefelsäure oder eine Sulfonsäure. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure.

Als Lewis-Säure kann beispielsweise Aluminiumtriflat eingesetzt werden.

In einer Ausführungsform beträgt die Menge an in Schritt c') zugesetzter Säure 0,3 bis 40 mol-%, bevorzugt 0,4 bis 15 mol-%, besonders bevorzugt 0,5 bis 5 mol-%, am meisten bevorzugt 0,6 bis 3 mol-%, bezogen auf die Stoffmenge der in Schritt a) eingesetzten ethylenisch ungesättigten Verbindung.

### Beispiele

Im Folgenden wird die Erfindung anhand von Ausführungsbeispielen näher beschrieben.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, und Pierre Granger, Pure Appl. Chem., 2001, 73, 1795-1818; Robin K. Harris, Edwin D. Becker, Sonia M. Cabral de Menezes, Pierre Granger, Roy E. Hoffman und Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Herstellung von Vorstufe G

### tert-Butylchlor(furan-2-yl)phosphin

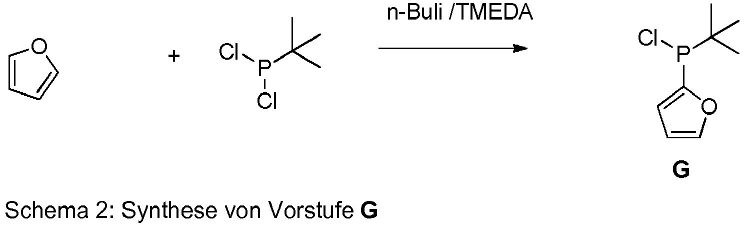

Chemikalienansatz: 1,6 ml Tetramethylethylendiamin (TMEDA) (1,05 eq, 10 mmol)
6 ml 1,6M n-Butyllithium (n-BuLi) (10 mmol, 1.05 eq)
1,5 g Dichlor(tert-butyl)phosphin (9,5 mmol)
0,7 ml Furan (9,7 mmol, 1,03 eq)
Absoluter Diethylether

0,64 g (0,7 ml, 9,4 mmol) Furan werden in einem 50 ml Dreihalskolben mit Thermometer und Tropftrichter unter Argon eingewogen und in 10 ml Diethylether gelöst. Dann gibt man 1,6 ml Tetramethylethylendiamin zu der Lösung. Das Gemisch wird dann auf-78 °C heruntergekühlt. Danach werden 6 ml 1,6 N n-Butyllithiumlösung in Hexan mittels Tropftrichter zugetropft. Den 50 ml Kolben mit der Reaktionsmischung lässt man anschließend 30 min bei Raumtemperatur rühren. Anschließend löst man 1,5 g tert- Butyldichlorphosphin in 20 ml Ether. Man tropft dann das Furan-n-BuLi-Gemisch bei -78 °C zur tert- Butyldichlorphosphinlösung. Danach wird auf Raumtemperatur erwärmt. Es fällt das Lithiumchlorid aus. Die Suspension wird filtriert und die Etherlösung im Vakuum bei 10⁻¹ Torr destilliert. Das Produkt ist ein farbloses Öl.
Ausbeute 0,75 g, 42%
Kp = 54 °C (10⁻¹ Torr)
Reinheit (NMR)= 100%,
³¹P NMR (CD₂Cl₂, 121 MHz)= 80,92 ppm,
¹³C NMR(CD₂Cl₂, 75 MHz) = 151,1 d, J_{PC} = 56 Hz, 148,56 s, 123,65 d, J_{PC} = 30,2 Hz, 111 d, J_{PC} = 7,2 Hz, 35,4 d, J_{PC} = 24,9 Hz, 25,9 d, J_{PC} = 18,3 Hz
¹H NMR (CD₂Cl₂, 300 MHz, d1 = 10 s): 7,63, dd, (J = 1,7 Hz, J = 0,7 Hz, 1H), 6,87 td (J = 2,5 Hz, J = 1 Hz, 1H), 6,38 dt (J = 4 Hz, J = 1,7 Hz, 1H), 1,1 (d, J = 14,8 Hz, 9 H)
GC MS (M/Z, I (%)): 190 (19), 155 (2,5), 133 (8,9), 99 (14), 69 (23,6), 57 (100), 41 (32,4)

### Herstellung von Vorstufe H:

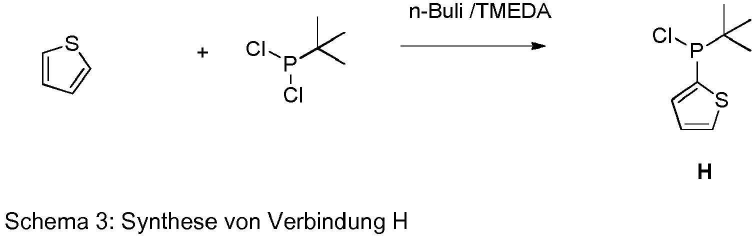

Chemikalienansatz: 2,5 ml TMEDA (16,6 mmol)
10ml 1,6N- Butyllithium(15,7 mmol)
2,5 g Dichlor(tert-butyl)phosphin
1,2 ml Thiophen
Absoluter Diethylether

1,2 ml Thiophen werden in einem 50 ml Dreihalskolben mit Thermometer und Tropftrichter unter Argon eingewogen und in 10 ml Diethylether gelöst. Dann gibt man 2,5 ml TMEDA zu der Lösung. Das Gemisch wird dann auf-78 °C heruntergekühlt. Danach werden 10 ml 1,6 N n-Butyllithiumlösung in Hexan mittels Tropftrichter zugetropft. Den 50 ml Kolben mit der Reaktionsmischung lässt man anschließend 30 min bei Raumtemperatur rühren. Anschließend löst man 2,5 g tert-Butyldichlorphosphin in 20 ml Ether. Man tropft dann das Thiophen-n-BuLi-Gemisch bei -78 °C zur tert-Butyldichlorphosphinlösung. Danach wird auf Raumtemperatur erwärmt. Die Etherlösung wird im Vakuum bei 10⁻¹ Torr destilliert. Das Produkt ist ein farbloses Öl.
Ausbeute 2,32 g, 70%
Kp = 54 °C (10⁻¹ Torr)
Reinheit (NMR)= 100%,
³¹P NMR (CD₂Cl₂, 121 MHz) = 99,8 ppm,
¹³C NMR(CD₂Cl₂, 75 MHz) = 1137,7 d, (J_{PC} = 59,9 Hz), 136,6 d (J_{PC} = 33 Hz), 133,1 s, 127,9 d (J_{PC} = 8,7 Hz), 35,1 d (J_{PC} = 28,1 Hz), 25,5 d (J_{PC} = 18,8 Hz), , ¹H NMR (CD₂Cl₂, 300 MHz, :7,59 dddd (J = 0,51, J = 1,1, J = 4,9, J = 6,0 1H), 7,34 dddd (J = 1,1, J = 3,5, J = 7,05, J = 10,6 1 H), 7,03, dddd, (J = 1,3, J = 3,5, J = 6,2 J = 8,4 1H), 1,0 d, (J = 14,7 Hz, 9 H)

### Herstellung von Verbindung 10 (Vergleichsverbindung)

Ausgehend von 1,1'-(Ferrocenediyl)phenylphosphin wird der gespannte Phosphanring mit PhLi geöffnet und die resultierende Zwischenstufe mit einem Chlorphosphin gequencht.

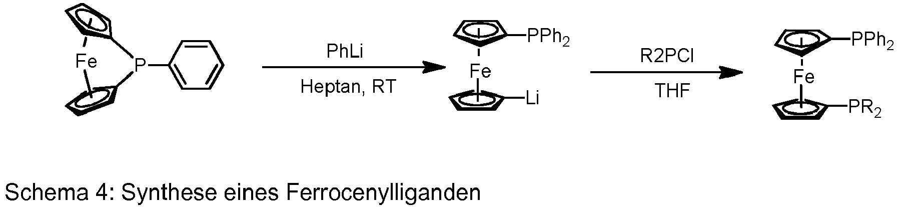

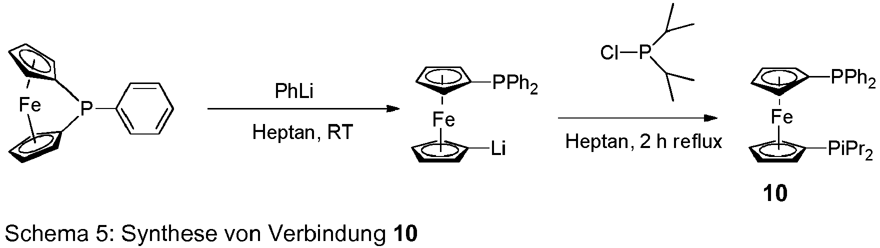

In einen 50 mL Rundkolben mit Magnetrührkern und Stickstoffansatz werden 1,13 mmol (565 µl) Phenyllithium (PhLi) vorgelegt und eine Lösung aus 1,03 mmol (300mg) cyclischen Phosphan in 20 ml Heptan langsam via Spritzenpumpe zugetropft. Das Li-Salz wird zweimal mit Heptan gewaschen und mit 6 ml Heptan versetzt. Zur Suspension wird eine Heptanlösung von 0,8 eq (0,824 mmol, 131 µl) ClP*i*Pr₂ in 7 ml Heptan bei Raumtemperatur zugetropft. Die rotbraune Suspension verfärbt sich kaum. Nach 20 min Rühren wird die Suspension unter Rückfluss 1,5 Stunden erwärmt. Der Feststoff hellt sich etwas auf. Lösungsmittel wird vollständig entfernt und der braun-rote Rückstand wird in H₂O und Ether aufgenommen. Die org. Phase wird zweimal mit H₂O gewaschen und über Na₂SO₄ getrocknet. Von der Etherphase wird ein ³¹P-Spektrum aufgenommen. Das Spektrum zeigt 2 Singuletts. Das Chlorphosphin ist vollständig verbraucht worden. Die Etherphase wird zur Trockene gebracht und man erhält 300 mg (Ausbeute: 61%) eines braungelben Öls, das sich in MeOH auf dem Wasserbad bei 65 °C gelöst. Die Lösung wird über Nacht in den Tiefkühler (-78 °C) gestellt. Es fallen 76 mg eines braungelben Öles aus, das NMR-spektroskopisch untersucht wird.
¹H NMR (300 MHz, CDCl₃) δ 7.46-7.23 (m, 10H, Ph), 4.36 (m, 2H, Cp), 4.21 (m, 2H, Cp), 34.24 (m, 4H, Cp), 1.88 (m, 2H, iPr), 1.15-0.96 (m, 12H, iPr).
¹³C NMR (75 MHz, CDCl₃) δ 139.9 (J = 9.8 Hz, Ph), 133.4 (J = 19.2 Hz, Ph), 128.4, 128.1, 128.0 (Ph), 77.1, 76.8, 76.2, 76.1 (Cp), 73.5 (J = 14.5 Hz, Cp), 72.8 (J = 2.9 Hz, Cp), 71.9 (J = 10.5 Hz, Cp),72.1 (Cp), 23.3 (J = 11.0 Hz, iPr), 20.1, 20.0, 19.9, 19.8 (iPr).
³¹P NMR (121 MHz, C₆D₆) δ = 0.88 und -16.62

### Herstellung von Verbindung 16

### 1,1' Bis(tert-butyl-2-furanyl)phosphino)ferrocen

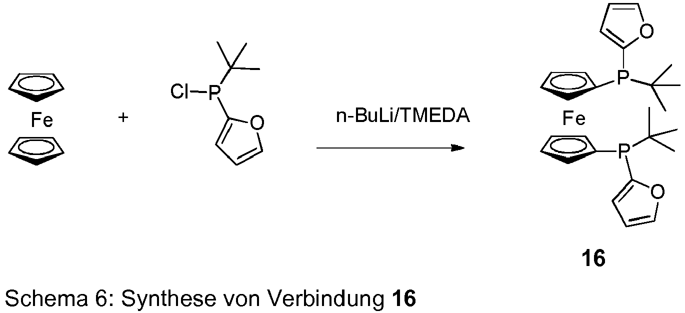

### Chemikalienansatz:

0,37 g Ferrocen (1,98 mmol)
2,2 ml TMEDA (Tetramethylethylendiamin) (7,34 mmol, 2,1 eq, 14,6 mmol)
10 ml 1,6N- Butyllithium (16 mmol, 2,28 eq)
0,75 g Chlor(tert-butyl-2-furanyl)phosphin (3,95 mmol)
Absoluter Diethylether, entgastes Wasser, Methanol unter Argon, Kieselgel G 60

In einem 50 ml Dreihalskolben versehen mit einem Magnetrührer werden 0,37 g Ferrocen unter Argon eingewogen und 5 ml absolutes Heptan zugegeben. Das Ferrocen löst sich vollständig. Danach werden 0,7 ml Tetramethylethylendiamin zur Lösung gegeben und anschließend 2,9 ml 1,6 N n-BuLi in Hexan zugesetzt. Die Reaktionslösung wird über Nacht bei Raumtemperatur kühl stehen gelassen. Es bildet sich ein Feststoff. Die überstehende Lösung wird entfernt. Zu dem Feststoff werden 10 ml Heptan gegeben. Dann werden 0,75 g tert-Butyl-chlor(furan-2-yl)phosphin in 5 ml absolutem THF gelöst und langsam zugetropft. Diese Lösung wird eine Stunde gerührt. Danach erfolgt ein Wechsel des Lösungsmittels von Heptan auf 10 ml Diethylether mittels Vakuum. Dann wird dreimal mit je 5 ml Wasser gewaschen. Die organische Phase wird über Na₂SO₄ (anhydrid) getrocknet. Die Lösung wird auf 10 ml eingeengt und eine Säulenchromatographie mit Ether durchgeführt. Anschließend wird Lösung eingeengt und aus heißem Methanol kristallisiert. Es fallen orange Kristalle aus. Es wird abdekantiert und getrocknet.
Ausbeute: 0,7 g
³¹P NMR (Aceton-d₆, 121 MHz) = 18,3 s, s, ppm,
¹³C NMR (Aceton-d₆, 75 MHz) = 147,9 s, 147,8 s, 123,4 d, J_{PC} = 34,3 Hz, 111,0 d, J_{PC} = 9 Hz, 78,1 d, J_{PC} = 42,6 Hz 74,5 s, 72,99 s, 72,4 d, J_{PC} = 9,5 Hz, 69,61 s, 31,70 d, J_{PC} = 7,3 Hz, 28,4 d, J_{PC} = 14,8 Hz.
¹H-NMR (Aceton-d₆, 300 MHz,): 7,85-7,8 m (2 H), 6,9-6,8 m (2H), 6,45-6,4 m (2 H), 4,5 m(1,3 H), 4,1 m (0,9 H), 3,9 m (1,3 H), 3,8 m (3,7 H), 2, 6 m (0,7 H), 0,8 (quint, J = 2,3 Hz, 18 H) HRMS (ESI) m/z⁺ berechnet für C₂₆H₃₂FeO₂P₂ (M+H)⁺ 495,13; gefunden 495,12983

### Herstellung von Verbindung 19 (Vergleichsverbindung)

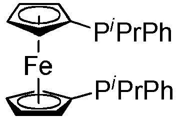

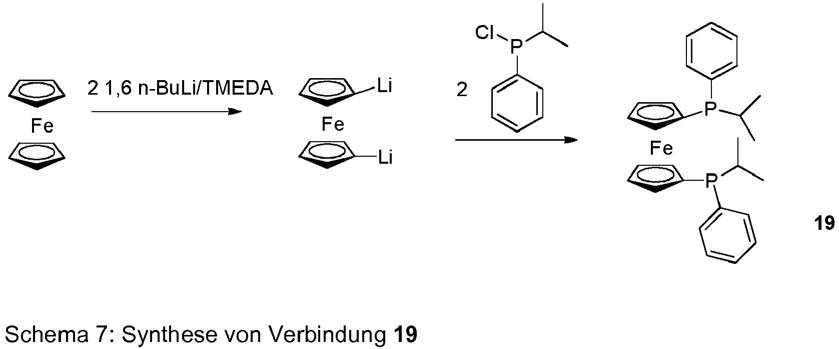

0,93 g Ferrocen werden in 50 ml absolutem Heptan in einem 100 ml Dreihalskolben, versehen mit einem Thermometer, Magnetrührer und Rückflusskühler, gelöst. Es werden 1,3 g TMEDA (1,6 ml) und 7,5 ml 1,6 n-BuLi/Hexan bei Raumtemperatur mittels Spritzen zugesetzt. Die Lösung wird 5 Stunden stehen gelassen. Es fallen große orangebraune Kristalle des dilithiierten Ferrocens aus. Die überstehende Lösung wird mittels einer Spritze entfernt. Und es werden 20 ml absolutes Heptan zugesetzt. Anschließend wird das Chlorphosphin gelöst in 10 ml Heptan zugetropft. Es wird ein Stunde unter Rückfluss erwärmt. Nach dem Abkühlen wird Die organische Phase dreimal mit jeweils 10 ml entgastem Wasser gewaschen. Es wird ins Trockene eingeengt und es werden 10 ml Diethylether zugesetzt. Diese Lösung wird mit Diethylether als Lösungsmittel durch 10 cm Kieselgel 60 unter Argon filtriert, eingeengt ins Trockene und aus wenig heißem Methanol kristallisiert das Zielprodukt in ca. 50 %-iger nicht optimierter Ausbeute.

### Analytik:

³¹P (121MHz, CDCl₃), - 7,8 s, - 8,15 s,
¹³C (75 MHz, CDCl₃);137,77, (d, J = 12 Hz), 137,4 (d, J = 11,3 Hz), 134,2 (d, J = 20,3 Hz), 129,1 s, 128,1 (d, J = 7,5 Hz), 77,4 (d, J = 11,3 Hz), 75,0 (d, J = 26,2 Hz), 74,0 (d, J = 22,3 Hz), 72,1 bs, 71,9-71,5 m, 71,1 s, 69,0 s, 27,6 (d, J = 10 Hz), 27,55 8d, J = 10 Hz), 20,3-19,9 m
¹H (300 MHz, CDCl₃): 7,52-7,44 (m, 4H), 7,33-7,23 (m, 6H), 4,23 (sept, J = 1,2 Hz, 1H), 4,1-4,0 (m, 4 H), 3,93-3,9 (m, 1H), 3,87-3,84 (m, 1H), 3,58-3,54 (m, 1H),
2,1-1,9 (m, 2 H), 0,99 (d, J = 7 Hz, 3H), 0,94 (d, J = 7 Hz, 3 H), 0,83-0.7 (m, 6H)

### Herstellung von Verbindung 22

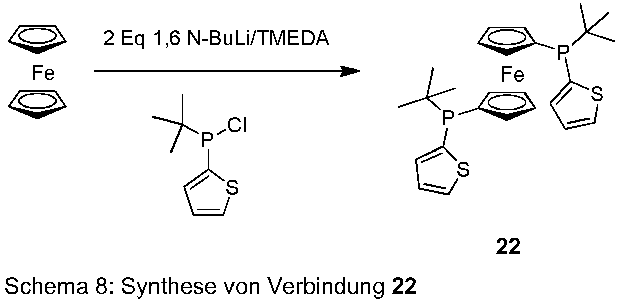

0,9 g tert.-Butylchlor(thiophen-2-yl)phosphan (4,36 mmol) werden unter Argon zusammen mit 5 ml Heptan in einem Tropftrichter eingewogen. In einen anderen 25 ml-Schlenkgefäß werden unter Argon 0,4 g Ferrocen (2,2 mmol) eingewogen, mit einem Magnetrührer versehen und mit 3 ml absolutem Heptan und 0,8 ml absolutem TMEDA (Tetramethylethylendiamin, 0,58 g, 5 mmol) versetzt. Es wird leicht erwärmt bis zum vollständigen Lösen des Ferrocens. Dann wird zur Ferrocenlösung bei Raumtemperatur 2,9 ml einer 1,6 N-Buthyllithiumlösung (4,6 mmol) zugesetzt. Dieses Schlenkgefäß wird 48 Stunden bei 4 °C im Kühlschrank stehen gelassen. Es bilden sich große Kristalle des Dilithiumsalzes des Ferrocens. (orangebraune Farbe). Von diesen wird die überstehende Lösung unter Argon abdekantiert. Und es werden 5 ml absolutes Heptan zugesetzt. Unter Rührung wird nun die Lösung mit dem Chlorphosphin zugesetzt und die Suspension wird eine Stunde bei Raumtemperatur gerührt. Die großen Kristalle lösen sich auf und ein Niederschlag aus gebildetem Lithiumchlorid ist zu beobachten. Dann wird diese Lösung dreimal mit jeweils 5 ml entgastem Wasser gewaschen. Es wird ins Trockene eingeengt und der Rückstand wird in 10 ml absoluten Diethylether aufgenommen. Diese Lösung wird mit Diethylether über 5 cm Kieselgel G 60 filtriert. Der Diethylether wird im Vakuum entfernt. Es bleibt ca. 1 g Rohprodukt übrig. Dieses wird mit 3 ml MeOH versetzt und im Kühlschrank bei 4 °C über Nacht stehengelassen. Es bilden sich orange Kristalle die in einer nicht optimierten Ausbeute von 500 mg als Zielprodukt erhalten werden (45% der Theorie).
Analytik: ³¹P (Aceton-d₆, 121 MHZ), -6,9 s, - 7,08 s
¹H(Aceton-d6, 300MHZ) 7,76-7,7 m (2H), 7,5-7,4 m (2H), 7,2 - 7,1 m (2H), 4,3 - 4,2 m(1,4 H), 4,13-4,08 m(0,7 H), 3,98-3,75 m, (5H), 0,8 d (J_{PH} = 12,8 Hz), 0,8 d (J_{PH}= 16,1 Hz),
¹³C (Aceton -d6 , 75 MHZ), 139.09 s, 138,6 d (J_{PC} = 7 Hz), 132,4 s, 127,8 d (J_{PC} = 10,5 Hz), 78,3 s, 77,8 s, 75,2 s, 73,6-73,3 m, 73,08 s, 72,6 d (J_{PC} = 9,6 Hz), 72,6 d (J_{PC} = 10 Hz), 69,7 s, 31,3 d (J_{PC} = 9,8 Hz), 28,1 d (J_{PC} = 15,3 Hz),
HRMS berechnet für C₂₆H₃₂Fe₁P₂S₂: 526,07646, gefunden: 526,07647,
MS(EI, 70eV(Mz/%), 526 (M+, 38), 469(100), 413(94), 329(5), 299(31), 266(6)216(18)171/17)151(4,58), 115 (8)

### Herstellung von Verbindung 34

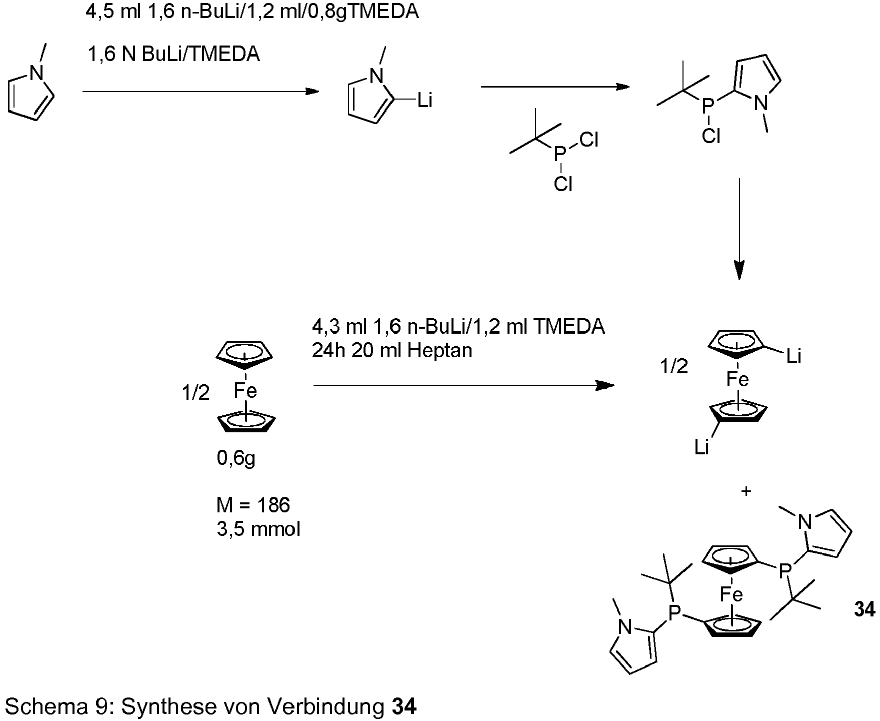

In einem 100 ml-Dreihalskolben versehen mit einem Magnetrührer und einem Tieftemperaturthermometer werden unter Argon 0,63 ml (0,547 g, 7,08 mmol) N-Methylpyrrol (frisch von Calciumhydrid destilliert), 20 ml absoluter Diethylether und 1,2 ml (0,8 g) TMEDA unter Rühren gemischt. Die Mischung wird auf -78 °C gekühlt und es werden mittels eines Tropftrichters innerhalb von 10 Minuten 4,35 ml 1,6 N BuLi Lösung in Hexan (7,2 mmol) zugetropft. Dann wird die Mischung auf Raumtemperatur erwärmt und eine halbe Stunde bei dieser Temperatur gerührt. Es werden 1,12 g Tert-butyldichlorphosphin in einem 100 ml-Schlenkgefäß unter Magnetrührung mit 20 ml absolutem Diethylether versetzt und auf -78 °C heruntergekühlt. Bei dieser Temperatur wird die erste Lösung bestehend aus Et₂O/TMEDA/lithiiertem N-Methylpyrrol unter Rühren der Lösung des Chlorphosphins zugesetzt. In einem weiteren 100 ml-Schlenkgefäß werden 0,65 g (3,5 mmol) Ferrocen in 10 ml Heptan unter Argon gelöst, mit 1,2 ml TMEDA (7,1 mmol) und 4,3 ml 1,6 N Butyllithiumlösung versetzt (7,1 mmol). Diese Lösung wird über Nacht bei 4 °C im Kühlschrank stehengelassen. Es bilden sich große orange Kristalle. Die überstehende Lösung wird abdekantiert und es werden 20 ml Heptan auf die Kristalle gegeben. Dann wird zu dieser gerührten Suspension bei Raumtemperatur die Lösung bestehend aus dem N-Methylimidazolyl-chlorphoshin mittels einer Kapillare zugesetzt. Diese Suspension wird eine Stunde bei Raumtemperatur gerührt. Dann wird dreimal mit 20 ml entgastem Wasser gewaschen. Anschließend wird im Vakuum bis ins Trockene eingeengt der ölige Rückstand in 20 ml absolutem Toluol aufgenommen und es wird unter Argon mit Toluol als Laufmittel über Kieselgel 60 gesäult. Ausbeute von 25 % (450 mg).
Analytische Daten
Reinheit (NMR)(100%)
³¹P NMR (CDCl₂, 121 MHz) = -27,41 s, -27,52 s
¹³C NMR (CDCl₂, 75 MHz) = 127,02 s, 125,34 s, 118,5 s, 108,11 s, 78,6 d, J_{PC} = 42 Hz,75,0 s, 72,7 d, J_{PC}= 6,3 Hz, 71,9 s, 71,4 d, J_{PC} = 11,5 Hz, 66,0 s, 36,2 d, J_{PC} = 21,9 Hz, 31,0 d, J_{PC} = 6,3 Hz, 27,75 d, J_{PC} = 15,8 Hz,
¹H NMR (CDCl₂, 300 MHz,): 6,82-6,7 m (2 H), 6,5, d,d J = 1,5 Hz, J = 3,7 Hz , 6,45 d,d J = 1,6, J = 3,7 Hz (2 H), 6,18 d,d, J = 2,5 Hz J= 3,7 Hz, 4,2 m, 4,1-3,9 m, 3,9 s, 3,8 s, 3,8-3,74 m, 0,8 d, J= 13.2 Hz
HRMS berechnet für C₂₈H₃₈FeN₂P₂: 520,18545, gefunden: 520,18643

### Hochdruckexperimente

### Einsatzstoffe:

Methanol (MeOH)
Ethen (auch als Ethylen bezeichnet)

Di-n-Buten wurde auch wie folgt bezeichnet: Dibuten, DNB oder DnB.

Di-n-Buten ist ein Isomerengemisch von C8-Olefinen, das aus der Dimerisierung von Gemischen aus 1-Buten, cis-2-Buten und trans-2-Buten entsteht. Technisch werden der Regel Raffinat II oder Raffinat III-Ströme einer katalytischen Oligomerisierung unterworfen, wobei die enthaltenen Butane (n/iso) unverändert hervorgehen und die enthaltenen Olefine ganz oder Teilweise umgesetzt werden. Neben dem dimeren Di-n-Buten, entstehen in der Regel auch höhere Oligomere (Tributen C12, Tetrabuten C16), die nach der Reaktion destillativ abgetrennt werden.

Ein industriell praktiziertes Verfahren zur Oligomerisierung von C4-Olefinen ist der sogenannte "OCTOL-Prozess".
Innerhalb der Patentliteratur beschreibt beispielsweise DE102008007081A1 eine auf dem OCTOL-Prozess beruhende Oligomerisierung. EP1029839A1 befasst sich mit der Fraktionierung der in dem OCTOL-Prozess entstehenden C8-Olefine.
Technisches Di-n-Buten besteht in der Regel 5 bis 30 % aus n-Octenen, 45 bis 75 % aus 3-Methylheptenen und zu 10 bis 35 % aus 3,4-Dimethylhexenen. Bevorzuge Ströme enthalten 10 bis 20 % n-Octene, 55 bis 65 % aus 3-Methylheptene und 15 bis 25 % 3,4-Dimethylhexenen.

Para-Toluolsulfonsäure wurde wie folgt abgekürzt: pTSA, PTSA oder p-TSA.
PTSA bezeichnet in diesem Text immer das para-Toluolsulfonsäure-Monohydrat.

### Allgemeine Vorschrift zur Durchführung der Hochdruckexperimente:

Allgemeine Versuchsbeschreibung für Reaktionen im Batchversuch:
Die entsprechenden Mengen an Substrat, Palladiumsalz, Säure und Alkohol werden unter Argon und Magnetrührung in einem 50 ml Schlenkgefäß vermischt.
Ein 100 ml Stahlautoklav der Firma Parr versehen mit einem Gaseinlass und einem Gasauslassventil, einem digitalem Druckaufnehmer, einem Temperaturfühler und einem Kugelhahn sowie einer eingebauten Kapillare zur Probennahme wird dreimal mittels Vakuum und Argonspülung von Sauerstoff befreit. Anschließend wird die Reaktionslösung aus dem Schlenkgefäß mittels einer Kapillare in den Autoklaven im Argongegenstrom durch den Kugelhahn befüllt. Anschließend wird entweder bei Raumtemperatur die entsprechende Menge an CO aufgepresst und dann auf Reaktionstemperatur hochgeheizt (Reaktionen, die nicht unter konstantem Druck gefahren werden) oder es wird erst auf Reaktionstemperatur hochgeheizt und dann wird das CO über eine Bürette, die mittels eines Druckminderers mit dem Autoklaven verbunden ist, aufgepresst. Diese Bürette wird dann noch auf ca. 100 bar mit CO befüllt und liefert während der Reaktion das benötigte CO bei einem konstanten Druck nach. Diese Bürette hat ein Totvolumen von ca. 30 ml und ist mit einem digitalen Druckaufnehmer versehen. Dann wird die Reaktion bei der benötigten Temperatur die entsprechende Zeit unter Rührung durchgeführt. Hierbei werden mittels einer Software (Specview der Firma SpecView Corporation) und einem Prozesscontroller der Firma Parr 4870 sowie einem 4875 Powercontroler Daten über den Druckverlauf im Autoklaven und in der Gasbürette aufgezeichnet. Falls benötigt werden über die Kapillare über die GC Proben gesammelt und analysiert. Hierzu wird vor der Reaktion eine geeignete exakte Menge (2-10 ml) Isooctan als interner Standard mit in das Schlenkgefäß gegeben. Diese geben auch Auskunft über den Reaktionsverlauf. Am Ende der Reaktion wird der Autoklav auf Raumtemperatur heruntergekühlt, der Druck vorsichtig abgelassen, falls nötig Isooctan als interner Standard hinzugefügt und eine GC-Analyse bzw. bei neuen Produkten auch eine GC MS Analyse durchgeführt.

Allgemeine Vorschrift für Versuche in den 12fach-Autoklaven (600 ml Parr-Autoklav):
In ausgeheizte Glasvials werden jeweils Di-n-Buten (DBN) und Methanol vorgelegt, mit einer Lösung aus Pd(acac)₂ (0,5 mg, 0,0016 mmol) und Ligand (0,0064 mmol) in 0,2 ml Methanol versetzt und H₂SO₄ (Lösung: 1 ml H₂SO₄ in 50 ml MeOH) zugegeben. Im Autoklaven werden die Ansätze 2-mal mit 10 bar CO gespült, mit dem gewünschten Druck CO beladen und bei der gewünschten Temperatur 20 h gerührt. Nach Beendigung der Reaktion werden jeweils Isooctan (interner Standard) und 1 ml EtOAc zugegeben. Die organische Phase wird per GC analysiert.

Die Ausbeuten der Reaktionen werden mittels GC (Isooctan als interner Standard) bestimmt.

### Analytik:

GC Analytik der Produkte aus Ethen: Für die GC Analyse wird ein Chromatograph der Firma Agilent GC Agilent 7890A mit einer 30 m HP Säule verwendet. Temperaturprofil: 35 °C, 10 min; 10 °C/min zu 200 °C, 16.5 min; das Injektionsvolumen beträgt 1 µl mit einem Split von 50:1. Retentionszeit von Methylpropionat : 6.158 min

GC Analytik Di-n-Buten: Für die GC Analyse wird ein Chromatograph der Firma Agilent GC Agilent 7890A mit einer 30 m HP5-Säule verwendet. Temperaturprofil: 35 °C, 10 min; 10 °C/min zu 200 °C; das Injektionsvolumen beträgt 1 µl mit einem Split von 50:1.
Retentionszeiten für Di-n-Buten und Produkte: 10.784-13.502 min
Die aus dem Di-n-Buten gebildeten Ester werden im Folgenden als MINO bezeichnet (Methylisononanoat).
Retentionszeit für Etherprodukte unbekannter Isomerenverteilung: 15.312, 17.042, 17.244, 17.417 min
Retentionszeit für iso-C9-Ester 19.502-20.439 min (Hauptpeak: 19.990 min)
Retentionszeit für n-C9-Ester: 20.669, 20.730, 20.884, 21.266 min.

### Analytik Methanol

Methanol wurde in einer Lösungsmitteltrocknungsanlage vorbehandelt: Pure Solv MD-/ Lösungsmittel Purification System, Firma Innovative Technology Inc. One Industrial Way, Amesbury MA 01013

### Wasserwerte:

Bestimmt mit Karl Fischer Tritration: TitraLab 580-TIM580, Firma Radiometer Analytical SAS (Karl- Fischer Titration), Wassergehalt: Messbereiche, 0,1-100% w/w, gemessener Wassergehalt: 0,13889%

### Eingesetzt wurden:

Technisches Methanol Applichem: Nr. A2954,5000, Chargennummer: LOT: 3L005446 Wassergehalt max. 1 %
Methanol Acros Organics (über Molsieb): Wassergehalt 0,005 %, Codenummer: 364390010, Chargennummer: LOT 1370321

TON: Turnovernumber, definiert als mol Produkt pro mol Katalysatormetall
TOF: Turnoverfrequenz, definiert als TON pro Zeit für das Erreichen eines bestimmten Umsatzes, z.B. 50 %

Das n/iso-Verhältnis gibt das Verhältnis von endständig zu Estern umgesetzten Olefinen zu innenständig zu Estern umgesetzten Olefinen an.

Die im Folgenden angegebenen n-Selektivitäten beziehen sich auf den Anteil der endständigen Methoxycarbonylierung bezogen auf die Gesamtausbeute an Methoxycarbonylierungsprodukten.

### Methoxycarbonylierung von Ethen mit Ligand 22

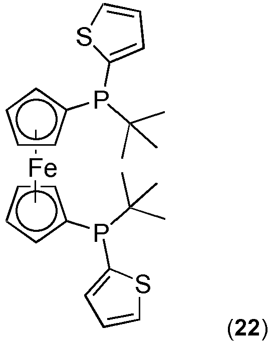

Ein 100 mLStahlautoklav wird mit Pd(acac)₂ (6.52 mg, 0.04 mol%) und Ligand **22** (45,5 mg, 0.16 mol%) und PTSA (61,1 mg, 0.6 mol%) und Methanol (20 mL) unter Argon befüllt. Dann werden 1.5 g, (53.6 mmol) Ethen (3.5 von Linde AG) in den Autoklaven überführt. (Massekontrolle des Autoklaven). Nach dem Aufheizen auf eine Reaktionstemperatur von 80 °C (Druck ungefähr 10 bar), wird bei dieser Temperatur CO (30 bar) aufgepresst. Bei dieser Temperatur wird die Reaktion 20 Stunden durchgeführt. Dann wird der Autoklav auf Raumtemperatur heruntergekühlt und entspannt. Der Inhalt wird in ein 50 ml Schlenkgefäß transferiert und Isooctan (interner Standard, 5.0 mL) wird zugesetzt. Die Ausbeute und Selektivität wurde mittels GC Analyse bestimmt. (Ausbeute: 91%).

### Methoxycarbonylierung von Ethen mit Ligand 59 (Vergleichsversuch)

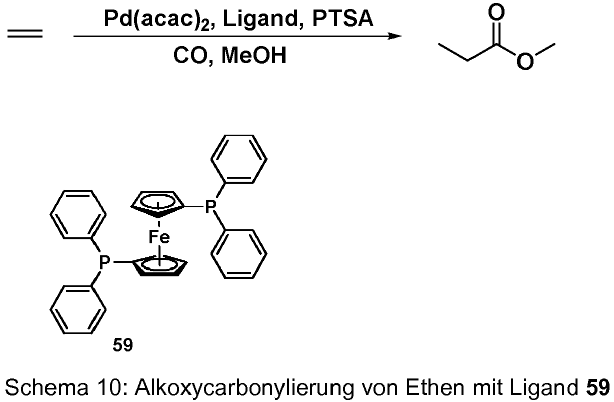

### Ligand 59:

Ligand **59,** 1,1'-Bis(diphenylphosphino)ferrocen, ist kommerziell verfügbar.

Ein 100 mL-Stahlautoklav wird mit Pd(acac)₂ (6.52 mg, 0.04 mol%) und Ligand **59** (47,9 mg, 0.16 mol%) und PTSA (61,1 mg, 0.6 mol%) und Methanol (20 mL) unter Argon befüllt. Dann werden 1.5 g, (53.6 mmol) Ethylen (3.5 von Linde AG) in den Autoklaven überführt. (Massekontrolle des Autoklaven). Nach dem Aufheizen auf eine Reaktionstemperatur von 80 °C (Druck ungefähr 10 bar), wird bei dieser Temperatur CO (30 bar) aufgepresst. Bei dieser Temperatur wird die Reaktion 20 Stunden durchgeführt. Dann wird der Autoklav auf Raumtemperatur heruntergekühlt und entspannt. Der Inhalt wird in ein 50 ml Schlenkgefäß transferiert und Isooctan (interner Standard, 5.0 mL) wird zugesetzt. Die Ausbeute und Selektivität wurde mittels GC Analyse bestimmt. (Ausbeute: 54%).

### Methoxycarbonylierung von Di-n-Buten mit Ligand 16

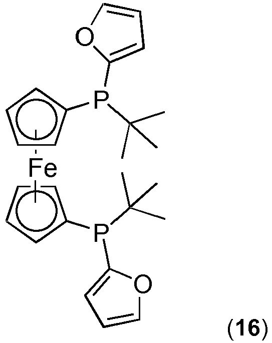

Ein 100 ml Stahlautoklav wird unter Argon mit Pd(acac)₂ (5,85 mg, 0.04 mol%), **16** (38 mg, 0.16 mol%), MeOH (20 mL), 7,54 mL Di-n-Buten (48 mmol) und PTSA (54,7 mg, 0.6 mol%) befüllt. Dann werden auf den Autoklaven 40 bar CO bei Raumtemperatur aufgepresst. Die Reaktion wurde 20 Stunden bei 120 °C durchgeführt. Nach der Reaktion wird der Autoklav auf Raumtemperatur heruntergekühlt und der Druck abgelassen. Es werden 5 ml Isooctan als interner Standard zur Lösung gegeben. Die Ausbeute und Selektivität wurde mittels GC-Analyse bestimmt. (Ausbeute: 30%, n/iso: 79:21)

### Methoxycarbonylierung von Di-n-Buten mit Liganden 10 und 19 (Vergleichsversuche im 12fach-Autoklaven)

Die Umsetzung von Di-n-Buten mit Hilfe von verschiedenen Liganden erfolgte nach folgender Vorschrift:
Ein 50 mL Schlenkgefäß wurde mit [Pd(acac)₂] (3.9 mg, 0.04 mol%), MeSO₃H (Methansulfonsäure) (13 uL, 0.6 mol%) und MeOH (20 mL) befüllt. Ein 4 mL-Fläschchen wurde mit Ligand X (0.16 mol%) befüllt und ein Magnetrührstab hinzugegeben. Danach wurden 1.25 mL der klaren gelben Lösung und Di-n-Buten (315 µL, 2mmol) mit einer Spritze injiziert. Das Fläschchen wurde auf einen Probenhalter gesetzt, der wiederum unter Argon-Atmosphäre in einen 600 mL Parr-Autoklaven eingesetzt wurde. Nach dreimaligem Spülen des Autoklaven mit Stickstoff wurde der CO-Druck auf 40 bar eingestellt. Die Reaktion lief bei 120 °C 20 Stunden. Nach Abschluss der Reaktion wurde der Autoklav auf Raumtemperatur abgekühlt und vorsichtig entspannt. Isooctan wurde als interner GC-Standard zugesetzt. Ausbeute und Regioselektivität wurden mittels GC bestimmt.

Die Ergebnisse sind im nachfolgenden Schema 11 zusammengefasst:

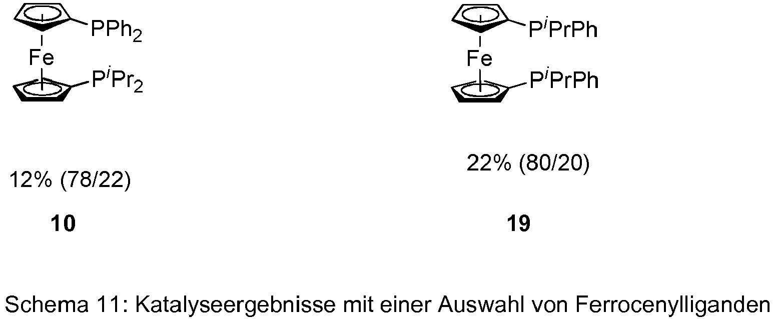

Die beschriebenen Experimente zeigen, dass sich die erfindungsgemäßen Verbindungen als Katalysatorliganden für die Alkoxycarbonylierung einer Vielzahl ethylenisch ungesättigter Verbindungen, insbesondere Ethen und Di-n-Buten, eignen. Insbesondere werden mit den erfindungsgemäßen Verbindungen bessere Ausbeuten erzielt als mit den aus dem Stand der Technik bekannten zweizähnigen Phosphinliganden, wie 1,1'-Bis(diphenylphosphino)ferrocen (Ligand **59**), 1-(Diphenylphosphino)-1'-(diisopropylphosphino)ferrocen (Ligand **10**) und 1,1'-Bis(isopropylphenylphosphino)ferrocen (Ligand **19**). Des Weiteren ermöglichen die erfindungsgemäßen Verbindungen auch die Alkoxycarbonylierung von technisch bedeutsamen, langkettigen Olefinen wie Di-n-Buten.

## Patentansprüche

1. Verbindung gemäß Formel (**I**) 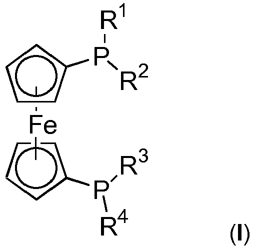 wobei
R¹ und R³ jeweils einen Heteroarylrest mit fünf Ringatomen darstellen,
R² und R⁴ jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl;
R¹ und R³ jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl,-CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl,-(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können;
und R² und R⁴, falls diese für -(C₁-C₁₂)-Alkyl, -(C₃-C₁₂)-Cycloalkyl, -(C₃-C₁₂)-Heterocycloalkyl oder -(C₆-C₂₀)-Aryl stehen,
jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl,-CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl,-(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können;
ausgenommen die Verbindungen gemäß den Formeln (**1**) und (**2**)

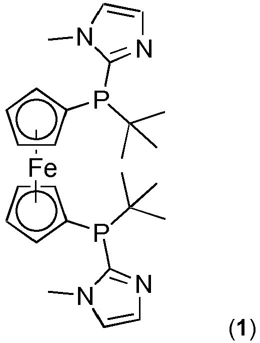

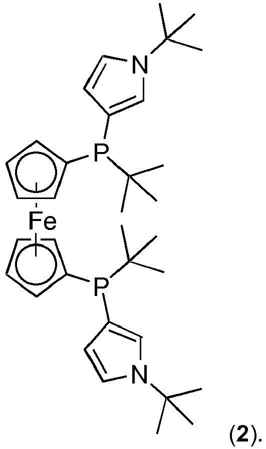

2. Verbindung nach Anspruch 1,
wobei R¹ und R³ jeweils unabhängig voneinander ausgewählt sind aus Furyl, Thienyl, 2-Pyrrolyl, 4-Imidazolyl, Oxazolyl, Isoxazolyl, Thiazolyl, Isothiazolyl, Pyrazolyl, Furazanyl, Tetrazolyl.

3. Verbindung nach einem der Ansprüche 1 bis 2,
wobei R¹ und R³ jeweils unabhängig voneinander ausgewählt sind aus Furyl und Thienyl.

4. Verbindung nach einem der Ansprüche 1 bis 3,
wobei R² und R⁴ jeweils unabhängig voneinander ausgewählt sind aus -(C₁-C₁₂)-Alkyl, -(C₃-C₁₂)-Cycloalkyl und -(C₆-C₂₀)-Aryl.

5. Verbindung nach einem der Ansprüche 1 bis 4,
wobei R² und R⁴ jeweils -(C₁-C₁₂)-Alkyl darstellen.

6. Verbindung nach einem der Ansprüche 1 bis 5,
wobei R¹ und R³ jeweils unabhängig voneinander mit einem oder mehreren Substituenten ausgewählt aus -(C₁-C₁₂)-Alkyl, -(C₃-C₁₂)-Cycloalkyl, -O-(C₁-C₁₂)-Alkyl, -O-(C₁-C₁₂)-Alkyl-(C₆-C₂₀)-Aryl,-O-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl substituiert sein können.

7. Verbindung nach einem der Ansprüche 1 bis 6,
wobei R³ und R⁴, falls diese für -(C₁-C₁₂)-Alkyl, -(C₃-C₁₂)-Cycloalkyl, -(C₃-C₁₂)-Heterocycloalkyl oder -(C₆-C₂₀)-Aryl stehen,
jeweils unabhängig voneinander mit einem oder mehreren Substituenten ausgewählt aus -(C₁-C₁₂)-Alkyl, -(C₃-C₁₂)-Cycloalkyl, -O-(C₁-C₁₂)-Alkyl, -O-(C₁-C₁₂)-Alkyl-(C₆-C₂₀)-Aryl,-O-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl substituiert sein können.

8. Verbindung nach einem der Ansprüche 1 bis 7,
gemäß einer der Formeln (**16**), (**22**) und (**34**)

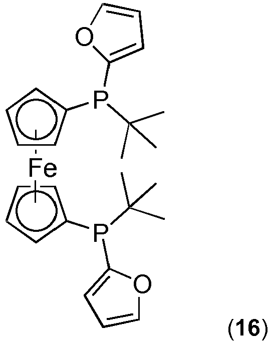

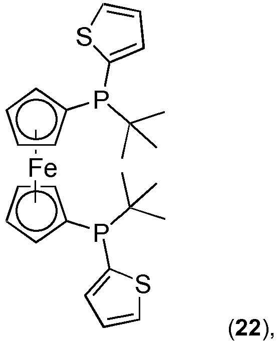

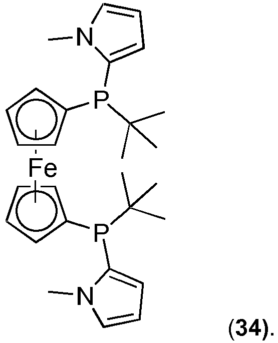

9. Komplex umfassend Pd und eine Verbindung nach einem der Ansprüche 1 bis 8.

10. Verfahren umfassend die Verfahrensschritte:
a) Vorlegen einer ethylenisch ungesättigten Verbindung;
b) Zugabe einer Verbindung nach einem der Ansprüche 1 bis 8 und einer Verbindung, welche Pd umfasst,
oder Zugabe eines Komplexes nach Anspruch 9;
c) Zugabe eines Alkohols;
d) Zuführen von CO;
e) Erwärmen des Reaktionsgemisches, wobei die ethylenisch ungesättigte Verbindung zu einem Ester umgesetzt wird.

11. Verfahren nach Anspruch 10,
wobei die ethylenisch ungesättigte Verbindung ausgewählt ist aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

12. Verfahren nach einem der Ansprüche 10 bis 11,
wobei die Verbindung, welche Pd umfasst, in Verfahrenschritt b) ausgewählt ist aus Palladiumdichlorid, Palladium(II)-acetylacetonat, Palladium(II)-acetat, Dichloro(1,5-cyclooctadiene)palladium(II), Bis(dibenzylideneaceton)palladium, Bis(acetonitrile)dichloropalladium(II), Palladium(cinnamyl)dichlorid.

13. Verfahren nach einem der Ansprüche 10 bis 12,
wobei der Alkohol in Verfahrensschritt c) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

14. Verwendung einer Verbindung gemäß einem der Ansprüche 1 bis 8 oder eines Komplexes gemäß Anspruch 9 zur Katalyse einer Alkoxycarbonylierungsreaktion.

## Claims

1. Compound of formula (**I**) 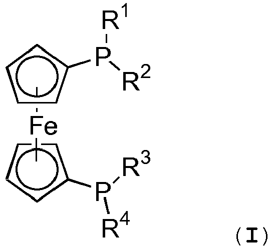 where
R¹ and R³ are each a heteroaryl radical having five ring atoms,
R² and R⁴ are each independently selected from -(C₁-C₁₂)-alkyl, -(C₃-C₁₂) -cycloalkyl, -(C₃-C₁₂) -heterocycloalkyl, -(C₆-C₂₀)-aryl;
R¹ and R³ may each independently be substituted by one or more substituents selected from -(C₁-C₁₂)-alkyl, -(C₃-C₁₂)-cycloalkyl, -(C₃-C₁₂)-heterocycloalkyl, -O-(C₁-C₁₂)-alkyl, -O-(C₁-C₁₂)-alkyl-(C₆-C₂₀)-aryl, -O-(C₃-C₁₂)-cycloalkyl, -S-(C₁-C₁₂)-alkyl, -S-(C₃-C₁₂)-cycloalkyl, -COO-(C₁-C₁₂)-alkyl, -COO-(C₃-C₁₂)-cycloalkyl, -CONH-(C₁-C₁₂)-alkyl, -CONH-(C₃-C₁₂)-cycloalkyl, -CO-(C₁-C₁₂)-alkyl, -CO-(C₃-C₁₂)-cycloalkyl, -N-[(C₁-C₁₂)-alkyl]₂, -(C₆-C₂₀)-aryl, -(C₆-C₂₀)-aryl-(C₁-C₁₂)-alkyl, -(C₆-C₂₀)-aryl-O-(C₁-C₁₂)-alkyl, -(C₃-C₂₀)-heteroaryl, -(C₃-C₂₀)-heteroaryl-(C₁-C₁₂)-alkyl, -(C₃-C₂₀)-heteroaryl-O-(C₁-C₁₂)-alkyl, -COOH, -OH, -SO₃H, -NH₂, halogen;
and R² and R⁴, if they are -(C₁-C₁₂)-alkyl, -(C₃-C₁₂)-cycloalkyl, -(C₃-C₁₂)-heterocycloalkyl or -(C₆-C₂₀)-aryl,
may each independently be substituted by one or more substituents selected from -(C₁-C₁₂)-alkyl, -(C₃-C₁₂)-cycloalkyl, -(C₃-C₁₂)-heterocycloalkyl, -O-(C₁-C₁₂)-alkyl, -O-(C₁-C₁₂)-alkyl-(C₆-C₂₀)-aryl, -O-(C₃-C₁₂)-cycloalkyl, -S-(C₁-C₁₂)-alkyl, -S-(C₃-C₁₂)-cycloalkyl, -COO-(C₁-C₁₂)-alkyl, -COO-(C₃-C₁₂)-cycloalkyl, -CONH-(C₁-C₁₂)-alkyl, -CONH-(C₃-C₁₂)-cycloalkyl, -CO-(C₁-C₁₂)-alkyl, -CO-(C₃-C₁₂)-cycloalkyl, -N-[(C₁-C₁₂)-alkyl]₂, -(C₆-C₂₀)-aryl, -(C₆-C₂₀)-aryl-(C₁-C₁₂)-alkyl, -(C₆-C₂₀)-aryl-O-(C₁-C₁₂)-alkyl, -(C₃-C₂₀)-heteroaryl, -(C₃-C₂₀)-heteroaryl-(C₁-C₁₂)-alkyl, -(C₃-C₂₀)-heteroaryl-O-(C₁-C₁₂)-alkyl, -COOH, -OH, -SO₃H, -NH₂, halogen;
excluding the compounds of the formulae (**1**) and (**2**)

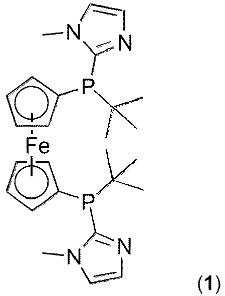

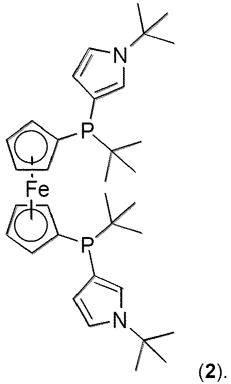

2. Compound according to Claim 1,
where R¹ and R³ are each independently selected from furyl, thienyl, 2-pyrrolyl, 4-imidazolyl, oxazolyl, isoxazolyl, thiazolyl, isothiazolyl, pyrazolyl, furazanyl, tetrazolyl.

3. Compound according to either of Claims 1 and 2,
where R¹ and R³ are each independently selected from furyl and thienyl.

4. Compound according to any of Claims 1 to 3,
where R² and R⁴ are each independently selected from -(C₁-C₁₂)-alkyl, -(C₃-C₁₂)-cycloalkyl and -(C₆-C₂₀)-aryl.

5. Compound according to any of Claims 1 to 4,
where R² and R⁴ are each -(C₁-C₁₂)-alkyl.

6. Compound according to any of Claims 1 to 5,
where R¹ and R³ may each independently be substituted by one or more substituents selected from -(C₁-C₁₂)-alkyl, -(C₃-C₁₂)-cycloalkyl, -O-(C₁-C₁₂)-alkyl, -O-(C₁-C₁₂)-alkyl- (C₆-C₂₀)-aryl, -O-(C₃-C₁₂)-cycloalkyl, -(C₆-C₂₀)-aryl, -(C₆-C₂₀)-aryl- (C₁-C₁₂)-alkyl, -(C₆-C₂₀)-aryl-O-(C₁-C₁₂)-alkyl.

7. Compound according to any of Claims 1 to 6,
where R³ and R⁴, if they are -(C₁-C₁₂)-alkyl, -(C₃-C₁₂)-cycloalkyl, -(C₃-C₁₂)-heterocycloalkyl or -(C₆-C₂₀)-aryl,
may each independently be substituted by one or more substituents selected from -(C₁-C₁₂)-alkyl, -(C₃-C₁₂)-cycloalkyl, -O-(C₁-C₁₂)-alkyl, -O-(C₁-C₁₂)-alkyl- (C₆-C₂₀)-aryl, -O-(C₃-C₁₂)-cycloalkyl, -(C₆-C₂₀)-aryl, -(C₆-C₂₀)-aryl- (C₁-C₁₂)-alkyl, -(C₆-C₂₀)-aryl-O-(C₁-C₁₂)-alkyl.

8. Compound according to any of Claims 1 to 7,
of one of the formulae (**16**), (**22**) and (**34**)

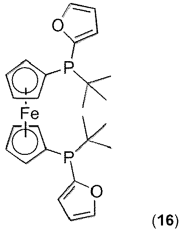

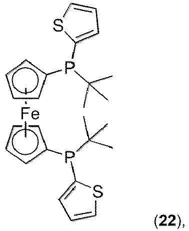

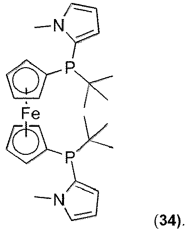

9. Complex comprising Pd and a compound according to any of Claims 1 to 8.

10. Process comprising the following process steps:
a) initially charging an ethylenically unsaturated compound;
b) adding a compound according to any of Claims 1 to 8 and a compound comprising Pd,
or adding a complex according to Claim 9;
c) adding an alcohol;
d) feeding in CO;
e) heating the reaction mixture, with conversion of the ethylenically unsaturated compound to an ester.

11. Process according to Claim 10,
wherein the ethylenically unsaturated compound is selected from ethene, propene, 1-butene, *cis-* and/or *trans*-2-butene, isobutene, 1,3-butadiene, 1-pentene, *cis-* and/or *trans*-2-pentene, 2-methyl-1-butene, 3-methyl-1-butene, 2-methyl-2-butene, hexene, tetramethylethylene, heptene, 1-octene, 2-octene, di-n-butene, and mixtures thereof.

12. Process according to either of Claims 10 and 11,
wherein the compound comprising Pd in process step b) is selected from palladium dichloride, palladium(II) acetylacetonate, palladium(II) acetate, dichloro(1,5-cyclooctadiene)palladium(II), bis(dibenzylideneacetone)palladium, bis(acetonitrile)dichloropalladium(II), palladium(cinnamyl) dichloride.

13. Process according to any of Claims 10 to 12,
wherein the alcohol in process step c) is selected from methanol, ethanol, 1-propanol, 1-butanol, 1-pentanol, 1-hexanol, 2-propanol, *tert*-butanol, 3-pentanol, cyclohexanol, phenol, and mixtures thereof.

14. Use of a compound according to any of Claims 1 to 8 or of a complex according to Claim 9 for catalysis of an alkoxycarbonylation reaction.

## Revendications

1. Composé selon la formule (I) 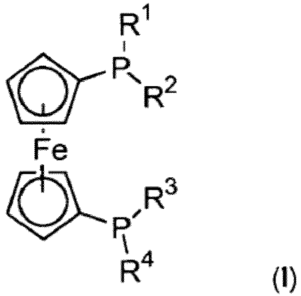 dans laquelle
R¹ et R³ représentent chacun un radical hétéroaryle contenant cinq atomes de cycle,
R² et R⁴ sont chacun choisis indépendamment l'un de l'autre parmi alkyle en (C₁-C₁₂), cycloalkyle en (C₃-C₁₂), hétérocycloalkyle en (C₃-C₁₂), aryle en (C₆-C₂₀) ;
R¹ et peuvent être substitués chacun indépendamment l'un de l'autre avec un ou plusieurs substituants choisis parmi :
alkyle en (C₁-C₁₂), cycloalkyle en (C₃-C₁₂), hétérocycloalkyle en (C₃-C₁₂), -O-alkyle en (C₁-C₁₂), -O-alkyle en (C₁-C₁₂)-aryle en (C₆-C₂₀), -O-cycloalkyle en (C₃-C₁₂), -S-alkyle en (C₁-C₁₂), -S-cycloalkyle en (C₃-C₁₂), -COO-alkyle en (C₁-C₁₂), -COO-cycloalkyle en (C₃-C₁₂),-CONH-alkyle en (C₁-C₁₂), -CONH-cycloalkyle en (C₃-C₁₂),-CO-alkyle en (C₁-C₁₂), -CO-cycloalkyle en (C₃-C₁₂), -N-[alkyle en (C₁-C₁₂)]₂, aryle en (C₆-C₂₀), aryle en (C₆-C₂₀)-alkyle en (C₁-C₁₂), aryle en (C₆-C₂₀)-O-alkyle en (C₁-C₁₂), hétéroaryle en (C₃-C₂₀), hétéroaryle en (C₃-C₂₀)-alkyle en (C₁-C₁₂), hétéroaryle en (C₃-C₂₀)-O-alkyle en (C₁-C₁₂),-COOH, -OH, -SO₃H, -NH₂, halogène ;
et R² et R⁴, si ceux-ci représentent alkyle en (C₁-C₁₂), cycloalkyle en (C₃-C₁₂), hétérocycloalkyle en (C₃-C₁₂) ou aryle en (C₆-C₂₀), peuvent être substitués chacun indépendamment l'un de l'autre avec un ou plusieurs substituants choisis parmi :
alkyle en (C₁-C₁₂), cycloalkyle en (C₃-C₁₂), hétérocycloalkyle en (C₃-C₁₂), -O-alkyle en (C₁-C₁₂), -O-alkyle en (C₁-C₁₂)-aryle en (C₆-C₂₀), -O-cycloalkyle en (C₃-C₁₂), -S-alkyle en (C₁-C₁₂), -S-cycloalkyle en (C₃-C₁₂), -COO-alkyle en (C₁-C₁₂), -COO-cycloalkyle en (C₃-C₁₂),-CONH-alkyle en (C₁-C₁₂), -CONH-cycloalkyle en (C₃-C₁₂),-CO-alkyle en (C₁-C₁₂), -CO-cycloalkyle en (C₃-C₁₂), -N-[alkyle en (C₁-C₁₂)]₂, aryle en (C₆-C₂₀), aryle en (C₆-C₂₀)-alkyle en (C₁-C₁₂), aryle en (C₆-C₂₀)-O-alkyle en (C₁-C₁₂), hétéroaryle en (C₃-C₂₀), hétéroaryle en (C₃-C₂₀)-alkyle en (C₁-C₁₂), hétéroaryle en (C₃-C₂₀)-O-alkyle en (C₁-C₁₂),-COOH, -OH, -SO₃H, -NH₂, halogène ;
à l'exception des composés selon les formules (1) et (2)

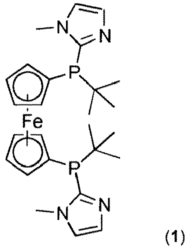

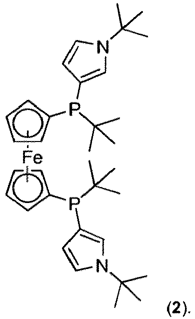

2. Composé selon la revendication 1, dans lequel R¹ et R³, sont chacun choisis indépendamment l'un de l'autre parmi furyle, thiényle, 2-pyrrole, 4-imidazolyle, oxazolyle, isoxazolyle, thiazolyle, isothiazolyle, pyrazolyle, furaznyle et tétrazolyle.

3. Composé selon l'une quelconque des revendications 1 à 2, dans lequel R¹ et R³ sont chacun choisis indépendamment l'un de l'autre parmi furyle et thiényle.

4. Composé selon l'une quelconque des revendications 1 à 3, dans lequel R² et R⁴ sont chacun choisis indépendamment l'un de l'autre parmi alkyle en (C₁-C₁₂), cycloalkyle en (C₃-C₁₂) et (C₆-C₂₀).

5. Composé selon l'une quelconque des revendications 1 à 4, dans lequel R² et R⁴ représentent chacun alkyle en (C₁-C₁₂).

6. Composé selon l'une quelconque des revendications 1 à 5, dans lequel R¹ et R³ peuvent chacun être substitués indépendamment l'un de l'autre avec un ou plusieurs substiuants choisis parmi :
alkyle en (C₁-C₁₂), cycloalkyle en (C₃-C₁₂), -O-alkyle en (C₁-C₁₂), -O-alkyle en (C₁-C₁₂)-aryle en (C₆-C₂₀), -O-cycloalkyle en (C₃-C₁₂), aryle en (C₆-C₂₀), aryle en (C₆-C₂₀) -alkyle en (C₁-C₁₂), aryle en (C₆-C₂₀)-O-alkyle en (C₁-C₁₂).

7. Composé selon l'une quelconque des revendications 1 à 6, dans lequel R³ et R⁴, si ceux-ci représentent alkyle en (C₁-C₁₂), cycloalkyle en (C₃-C₁₂), hétérocycloalkyle en (C₃-C₁₂) ou aryle en (C₆-C₂₀), peuvent chacun être substitués indépendamment l'un de l'autre avec un ou plusieurs substituants choisis parmi :
un alkyle en (C₁-C₁₂), cycloalkyle en (C₃-C₁₂), -O-alkyle en (C₁-C₁₂), -O-alkyle en (C₁-C₁₂)-aryle en (C₆-C₂₀), -O-cycloalkyle en (C₃-C₁₂), aryle en (C₆-C₂₀), aryle en (C₆-C₂₀) -alkyle en (C₁-C₁₂), aryle en (C₆-C₂₀)-O-alkyle en (C₁-C₁₂).

8. Composé selon l'une quelconque des revendications 1 à 7, selon l'une quelconque des formules (16), (22) et (34)

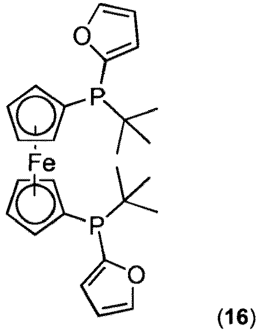

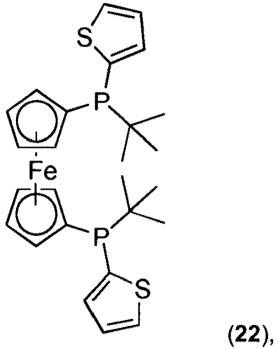

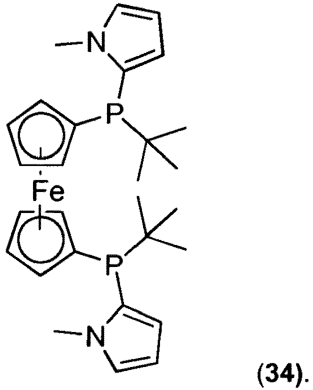

9. Complexe comprenant Pd et un composé selon l'une quelconque des revendications 1 à 8.

10. Procédé comprenant les étapes de procédé suivantes :
a) le chargement d'un composé éthyléniquement insaturé ;
b) l'ajout d'un composé selon l'une quelconque des revendications 1 à 8 et d'un composé qui comprend Pd,
ou l'ajout d'un complexe selon la revendication 9 ;
c) l'ajout d'un alcool ;
d) l'introduction de CO ;
e) le chauffage du mélange réactionnel, le composé éthyléniquement insaturé étant transformé en un ester.

11. Procédé selon la revendication 10, dans lequel le composé éthyléniquement insaturé est choisi parmi l'éthène, le propène, le 1-butène, le *cis-* et/ou le *trans*-2-butène, l'iso-butène, le 1,3-butadiène, le 1-pentène, le *cis-* et/ou le *trans*-2-pentène, le 2-méthyl-1-butène, le 3-méthyl-1-butène, le 2-méthyl-2-butène, l'hexène, le tétraméthyléthylène, l'heptène, le 1-octène, le 2-octène, le di-n-butène ou leurs mélanges.

12. Procédé selon l'une quelconque des revendications 10 à 11, dans lequel le composé qui comprend Pd à l'étape de procédé b) est choisi parmi le dichlorure de palladium, l'acétylacétonate de palladium (II), l'acétate de palladium (II), le dichloro(1,5-cyclooctadiène)palladium (II), le bis(dibenzylidène-acétone)palladium, le bis(acétonitrile)dichloropalladium (II), le dichlorure de palladium(cinnamyle).

13. Procédé selon l'une quelconque des revendications 10 à 12, dans lequel l'alcool à l'étape de procédé c) est choisi parmi le méthanol, l'éthanol, le 1-propanol, le 1-butanol, le 1-pentanol, le 1-hexanol, le 2-propanol, le *tert*-butanol, le 3-pentanol, le cyclohexanol, le phénol ou leurs mélanges.

14. Utilisation d'un composé selon l'une quelconque des revendications 1 à 8 ou d'un complexe selon la revendication 9 pour la catalyse d'une réaction d'alcoxycarbonylation.
